(19) Europäisches Patentamt
European Patent Office
Office européen des brevets

(11) EP 2 759 303 A2

(12) EUROPEAN PATENT APPLICATION
published in accordance with Art. 153(4) EPC

(43) Date of publication:
30.07.2014 Bulletin 2014/31

(51) Int Cl.:
A61K 38/14 (2006.01)    A61K 36/258 (2006.01)
A61P 25/28 (2006.01)    A61P 25/00 (2006.01)

(21) Application number: 12834061.9

(22) Date of filing: 19.09.2012

(86) International application number:
PCT/KR2012/007505

(87) International publication number:
WO 2013/042943 (28.03.2013 Gazette 2013/13)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 19.09.2011   KR 20110094194
14.09.2012   KR 20120102419

(71) Applicant: Konkuk University Industrial
Cooperation Corp.
Seoul 143-729 (KR)

(72) Inventors:
• NAH, Seung Yeol.
Seoul 135-230 (KR)
• HWANG, Sung Hee.
Gyeonggi-Do 431-080 (KR)
• SHIN, Tae Joon.
Seoul 137-070 (KR)
• CHOI, Sun Hye.
Seoul 134-872 (KR)

(74) Representative: Nevant, Marc et al
Cabinet Beau de Loménie
158, rue de l'Université
75340 Paris Cedex 07 (FR)

(54) USE FOR GLYCOLIPOPROTEIN GINTONIN, ISOLATED AND IDENTIFIED FROM GINSENG, AS A NATURAL MEDICINAL-PLANT-DERIVED LIGAND

(57) The present invention relates to glycolipoprotein gintonin, isolated and identified from ginseng, as a natural medicinal-plant-derived ligand acting on LPA1(lysophosphatidic acid; 1- or 2-acyl-sn-glycerol-3-phosphate), LPA2, LPA3, LPA4 and LPA5 receptors whose efficacy is exhibited physiologically/pharmaceutically via an interaction with subset receptors [LPA1(edg-2), LPA2 (edg-4), LPA3(edg-7), LPA4, PLA5] in the EDG (endothelial differentiation gene) family in G protein-coupled receptors (GPCRs) present in the cell membranes of animals including humans. The gintonin of the present invention can be used to advantage in the prevention and treatment of various diseases arising from reduced calcium concentration and various physiological activities and pharmaceutical activities dependent on calcium, since the gintonin of the present invention interacts with LPA receptors so as to activate a series of signal transmission processes and temporarily induce an increase in free $Ca^{2+}$ in the cytoplasm, and a temporary increase in the intracellular calcium concentration gives rise to a temporary increase in the intracellular calcium concentration in various organs including, inter alia, those of the nervous system, cardiovascular system, endocrine system, reproductive system, digestive system and immune system where the LPA receptors are expressed, with physiological activity being exhibited.

FIG. 3

<110>    KONKUK UNIVERSITY INDUSTRIAL COOPERATION CORP.

<120>    USE OF A GINSENG GINTONIN AS HERBAL MEDICINE-DERIVED LIGAND

<130>    PP-E0541

<150>    KR 10-2011-0094194
<151>    2011-09-19

<150>    KR 10-2012-0102419
<151>    2012-09-14

<160>    11

<170>    KopatentIn 2.0

<210>    1
<211>    10
<212>    PRT
<213>    Ginseng

<400>    1
Arg Asp Ile Glu Ala His His Leu Pro Lys
 1               5               10


<210>    2
<211>    16
<212>    PRT
<213>    Ginseng

<400>    2
Lys Asp Pro Thr Ser Tyr Leu Asp Phe Leu Leu Ser Val Thr Arg Asp
 1               5               10              15



<210>    3
<211>    13
<212>    PRT
<213>    Ginseng

<400>    3
Lys Glu Glu Ile Val Ala Ile Asp Glu Glu Asp Lys Ser
 1               5               10


<210>    4
<211>    8
<212>    PRT
<213>    Ginseng

<400>    4
Lys Leu Asn Glu Ser Val Lys Asp
 1               5


<210>    5
<211>    151
<212>    PRT
<213>    Ginseng

<400>     5
Met Gly Leu Thr Gly Lys Leu Ile Cys Gln Thr Gly Ile Lys Ser Asp
  1               5                  10                  15

Gly Asp Val Phe His Glu Leu Phe Gly Thr Arg Pro His His Val Pro
            20                  25                  30

Asn Ile Thr Pro Ala Asn Ile Gln Gly Cys Asp Leu His Glu Gly Glu
            35                  40                  45

Phe Gly Lys Val Gly Ser Val Val Ile Trp Asn Tyr Ser Ile Asp Gly
        50                  55                  60

Asn Ala Met Ile Ala Lys Glu Glu Ile Val Ala Ile Asp Glu Glu Asp
65                  70                  75                  80

Lys Ser Val Thr Phe Lys Val Val Glu Gly His Leu Phe Glu Glu Phe
                85                  90                  95

Lys Ser Ile Val Phe Ser Val His Val Asp Thr Lys Gly Glu Asp Asn
            100                 105                 110

Leu Val Thr Trp Ser Ile Asp Tyr Glu Lys Leu Asn Glu Ser Val Lys
            115                 120                 125

Asp Pro Thr Ser Tyr Leu Asp Phe Leu Leu Ser Val Thr Arg Asp Ile
    130                 135                 140

Glu Ala His His Leu Pro Lys
145                 150


<210>     6
<211>     8
<212>     PRT
<213>     Ginseng

<400>     6
Arg Ser Asp Tyr Pro Trp Ala Met
  1               5


<210>     7
<211>     25
<212>     PRT
<213>     Ginseng

<400>     7
Lys Ala Phe Asp Ile Val Gly Leu Leu Asn Gln Glu Gly Ile Tyr Pro
  1               5                  10                  15

Asn Asn Asp Leu Tyr Arg Pro Lys Met
            20                  25


<210>     8
<211>     19
<212>     PRT
<213>     Ginseng

<400>     8
Lys Ser Leu Leu Asn Thr Phe Thr Ile His Gly Leu Tyr Pro Tyr Asn
  1               5                  10                  15

26

Ala Lys Gly

```
<210>    9
<211>    14
<212>    PRT
<213>    Ginseng

<400>    9
Lys His Leu Asn Ala Val Pro Glu Ile Asp Phe Thr Lys Asn
  1               5                   10


<210>    10
<211>    8
<212>    PRT
<213>    Ginseng

<400>    10
Arg Thr Ala Leu Ala Phe Arg Lys
  1               5


<210>    11
<211>    238
<212>    PRT
<213>    Ginseng

<400>    11
Met Arg Ala Ile Tyr Ile Ile Ser Val Ile Ile Val Ser Leu Ser Ile
  1               5                   10                  15

Phe Ser Trp Gly Gly Asn Ala Arg Ser Asp Tyr Pro Trp Ala Met Phe
             20                  25                  30

Ala Leu Arg Leu Gln Trp Pro Ala Gly Phe Cys Glu Val Asn Asn Ala
             35                  40                  45

Cys Asp Thr Lys Ser Leu Leu Asn Thr Phe Thr Ile His Gly Leu Tyr
         50                  55                  60

Pro Tyr Asn Ala Lys Gly Thr Pro Ala Leu Tyr Cys Asp Gly Thr Ala
     65                  70                  75                  80

Phe Asp Val Asn Ser Val Ser Asp Phe Leu Ala Glu Met His Leu Ala
                 85                  90                  95

Trp Pro Ser His Glu Thr Asn Thr Glu Asp Ile Gln Phe Trp Glu His
             100                 105                 110

Glu Trp Lys Lys His Gly Arg Cys Ser Glu Ala Leu Leu Lys Gln Thr
         115                 120                 125

Asp Tyr Phe Arg Thr Ala Leu Ala Phe Arg Lys Ala Phe Asp Ile Val
         130                 135                 140

Gly Leu Leu Asn Gln Glu Gly Ile Tyr Pro Asn Asn Asp Leu Tyr Arg
145                 150                 155                 160

Pro Lys Met Ile Lys Glu Ala Ile Lys Lys His Leu Asn Ala Val Pro
                 165                 170                 175
```

```
Glu Ile Asp Phe Thr Lys Asn Glu Asn Ser Glu Tyr Val Leu Thr Asp
        180                     185                 190

Ile Asn Val Cys Val Asn Gln Gln Ala Thr Arg Phe Val Asp Cys Pro
        195                     200                 205

Thr Asp Asp Ala Thr Asp Asp Tyr Arg Leu Lys Phe Val Arg Leu Pro
    210                 215                 220

Ser Lys Met Lys Phe Ala Asp Pro Arg Thr Asn Ser Ile Ile
225                 230                 235
```

**Description**

**Technical Field**

The present invention relates to the novel use of the glycolipoprotein gintonin isolated and identified from ginseng. More particularly, the present invention relates to the ginseng-derived glycolipoprotein gintonin which functions as a ligand to lysophosphatidic acid receptors endogenous to animals and humans, and as an agent for the prevention and treatment of LPA receptor-related diseases.

**Background Art**

From old times, ginseng has been taken orally as a general tonic or an adaptogenic agent for many purposes, inter alia, for the promotion of longevity and the enhancement of bodily functions against stress, fatigue, diseases, cancer and diabetes. Such a pharmaceutical belief has led people for hundreds of years in Korea, China and Japan to ingest ginseng. Currently, ginseng is one of the most famous and precious herbal medicines consumed around the world (Tyler, J. Pharm. Technol.11, 214-220, 1995).

Ginsenosides have typically been utilized in many physiological and pharmaceutical studies from the beginning of the 1960s in which they were first isolated and reported as representative ingredients of ginseng (Shibata, et al. Tetraheadron Letters 1962, 1239-1245, 1963; Wagner-Jauregg and Roth, Pharm Acta Helv 37, 352-357, 1962). In addition, ginseng was also found to contain other ingredients including polysaccharides, polyacetylenes, and proteins (Nah, Kor. J. Ginseng Sci. 21, 1-12, 1997).

Previously, the present inventors succeeded in isolating and identifying novel glycolipoproteins, consisting composed of saccharides, lipids and proteins, from ginseng, and designated them gintonin. Apart from well-known ginseng saponins (ginsenosides), gintonin was found to exert its action through a membrane signal transduction pathway triggered by interaction with a membrane protein which has not yet identified. For example, gintonin induces a transient increase in cytoplasmic free $Ca^{2+}$ level in mouse EAT (Ehrlich Ascites Tumor) through a phospholipase C (PLC)-coupled signaling pathway, like a signaling pathway functioning upon the activation of G protein-coupled receptors (GPCRs or 7TM receptors). In addition, gintonin was found to activate endogenous $Ca^{2+}$-activated $Cl^-$ channel (CaCC) through the signaling pathway that activates PTX-insensitive G protein→PLC→$IP_3$→calcium release from the calcium reservoir endoplasmic reticulum (ER) in *Xenopus* oocytes (Pyo et al., J Ginseng Res, 35, 92-103, 2011).

As a 2nd messenger, intracellular calcium ($Ca^{2+}$) plays a pivotal role in various physiological functions in animals including humans. For example, it is involved in a variety of cellular functions including the regulation of membrane excitability, the release and secretion of neurotransmitters/hormones, karyokinesis, synaptic plasticity, and calcium-dependent enzymatic activation and ion channel regulation. When occurring, an intracellular global $Ca^{2+}$ wave is responsible for fertilization, contraction of various muscles, such as smooth muscle, skeletal muscle, cardiac muscle, etc., hepatic metabolism, gene transcription, and cell proliferation.

In addition, if individual $Ca^{2+}$ channels of neighboring cells are connected, an intercellular global $Ca^{2+}$ wave is produced which is responsible for wound healing, bile juice/insulin release, and nitric oxide (NO) synthesis (Berridge et al., Natuare 395. 645-648, 1998).

Intracellular supply of calcium is achieved mainly by two routes. First, membrane excitability in excitatory cells, such as membrane depolarization, activates voltage-gated $Ca^{2+}$ channels through which calcium influx is then induced. Next, when an extracellular GPCR ligand binds to a corresponding GPCR, intracellular free $Ca^{2+}$ levels are increased through the pathway G proteins (generally, $G\alpha_{q/11}$ proteins)→PLC→$IP_3$ receptor→ER $Ca^{2+}$ reservoir→$Ca^{2+}$ release (Berridge et al., Natuare 395. 645-648, 1998).

The transient increase of intracellular free $Ca^{2+}$ levels induced by binding ligands, such as neurotransmitters and hormones, to membrane G protein-coupled receptors is generally mediated through the signaling pathway $G\alpha_{q/11}$ proteins→PLC→$IP_3$ receptor→ER $Ca^{2+}$ reservoir→$Ca^{2+}$ release (Berridge et al., Nature 395. 645-648, 1998).

As stated above, gintonin induces animal cells to increase in intracellular free $Ca^{2+}$ level through the $G\alpha_{q/11}$ protein→PLC→$IP_3$ receptor→ER Ca2+ reservoir→$Ca^{2+}$ release signal pathway, but the membrane protein (receptor) which interacts with gintonin to increase intracellular free $Ca^{2+}$ has not yet been known.

Glycerol- and sphingosine-based phospholipids are abundantly found in cell membrane, serving as structural components of cell membranes. The phospholipids are also found in blood. Some of them are metabolized into lysophospholipids (Okudaira et al., Biochimie 92, 698-706, 2010).

For example, lysophosphatidylcholine (LPC), a kind of lisophospholipids, is degraded to lysophosphatidic acid (LPA; 1-or 2-acyl-sn-glycerol-3-phosphate) by lisophospholipase D, known as autotoxin (Aoki, Seminars Cell & Dev. Biol. 15, 477-489, 2004; Okudaira et al., Biochimie 92, 698-706. 2010). Early studies reported that LPA may be involved in hemostasis, wound healing, and tissue regeneration since it was found to be produced upon platelet activation. However, recent studies showed that LPA exists in an amount of 1-5 $\mu$M in plasma, serum, saliva, seminal fluid, follicular fluid, in addition to platelets (Aoki, Seminars Cell & Dev. Biol. 15, 477-489, 2004). Further, LPA are now found to be distributed over a wide spectrum of cells including adipose cells, fibroblast, brain cells, and other organ cells (Pages et al., Prostaglandins 64, 1-10, 2001).

LPA present in blood becomes more stable when associating with plasma proteins (e.g., albumin). During circulation, LPA associated with a plasma protein (e.g., albumin) binds to an LPA receptor in a target organ to exert its action (Croset et al., Biochem J 345, 61-67, 2000). LPA activity is accounted for predominantly by the phosphate group and glycerol backbone of LPA (Jalink et al., Biochem J. 307, 609-615, 1995). LPA in blood is apt to be inactivated because LPA, although present in an amount of 1~5 $\mu$M, is degraded within a very short period of time by free or membrane-bound lysophospholipid phosphatase to delete the phosphate group therefrom. For this reason, extensive research has been conducted to synthesize or develop long-acting LPA analogs that are less prone to enzymatic degradation, but compounds suitable for use in clinical application have not yet been discovered (Pilquil et al., Prostaglandins. 64, 83-92, 2001; Brindley and Pilquil, J Lipid Res. 50 SupplS225-230, 2009; Croset et al., Biochem J 345. 61-67, 2000; Deng et al., Gastroenterology 132. 1834-1851, 2007).

Lysophosphatidic acid (LPA) serves as a second messenger in the cytoplasm. However, what is more important, this simple lipid interacts with membrane proteins (extracellular receptor) of vertebrates, playing a role in physiological/pharmaceutical functions within cells. A receptor to which LPA binds was first cloned from the ventricular zone where neural cells are nascent in the cerebrum in the embryonic phase (Hecht et al., 135, 1071-1083, 1996).

The LPA receptor is a member of the G protein-coupled receptors (GPCRs). The LPA receptor was first categorized into the endothelial differentiation gene (EDG) family since it shares high homology with both DNA and protein sequences of EDG family receptors. Recent studies classified the LPA receptor as subfamilies of LPA and S1P receptors (Chun J et al., Pharmacol Rev. 62, 579-587, 2010). The LPA receptor were also observed to consist of 6 subtypes (Chun J et al., Pharmacol Rev. 62, 579-587, 2010).

Currently, the LPA receptor is classified into LPA1-LPA6 receptors. Particularly, LPA1 and LPA2 receptors are found in abundance in the brain, and are implicated in brain development such as gyrus development and neurogenesis (An et al., J Biol Chem 283, 7906-7910, 1998).

Compared to GPCRs, other LPA receptors including LPA1 and LPA2 are widely distributed over almost all organs from the nascent brain to mature organs of vertebrates including humans. These LPA receptors exert diverse physiological and pharmaceutical actions in almost all organs (cardiovascular, nerve, endocrine, reproductive, and immune systems) (Skoura and Hla, J Lipid Res. 50, S293-S298, 2009).

In detail, when binding to each of LPA1-LPA6 receptors, members of GPCRs, LPA is known to modulate differentiation, migration and motility, morphology change, proliferation, survival, antiogenesis, inflammation, platelet aggregation and other diverse cellular physiological/pharmaceutical activities (Toman and Spiegel, Neurochemical Research 27, 619-627, 2002; Gardall et al., 2006).

Since they accounts for diverse physiological activities in a variety of organs, the LPA receptors are regarded as important targets of LPA receptor-related drugs. However, no ligands acting LPA receptors (particularly, ligands derived from plants) have been reported, thus far, except for LPA, which is found in blood and cytoplasm of vertebrates and is prone to inactivation by enzymatic metabolism/degradation (Tigyi, Br J Pharmacol 161, 241-270, 2010).

One of the most important common features of these receptors is that when LPA binds to each of LPA1-LPA6 receptors, they couple with various kinds of GTP-binding proteins ($G\alpha_{i/o}$, $G\alpha_{q/11}$, $G\alpha_{12/13}$ and $G\alpha_s$) which, in turn, exhibit a variety of biological activities in conjuction with various effector systems (Yoshida and Ueda, Jpn J Pharmacol 87, 104-109, 2001).

By way of example, PLC is activated to induce an increase in intracellular free $Ca^{2+}$ (Toman and Spiegel, 2002; Dubbin et al., J Neurosci 30, 7300-7309, 2010). As mentioned above, intracellular free $Ca^{2+}$ serves as a second messenger responsible for a variety of life signals covering various enzymatic activities and gene description (Berridge et al., Nature 395. 645-648, 1998). The various biological activities according to the activation of the LPA receptors by the LPA ligand are considered to be accounted for by the increased level of intracellular free $Ca^{2+}$. When activated, the LPA6 receptor, however, is reported to induce adenylate cyclase activity rather than the increase of intracellular free $Ca^{2+}$ (Yanagida et al., J Biol Chem 284, 17731-17741, 2009).

Experiments with LPA receptor-knockout animals showed that LPA receptors have important influence on the development of various organs including fetal brain. To quote an example, LPA1 receptor-knockout animals gave a stillbirth due to abnormal encephalization (Choi et al., Biochim Biophys Acta 1781, 531-539, 2008). For genital organs, LPA1 receptor-knockout female animals are reported to well nidate, but reduce the number of embryos. Other various side effects are also reported. LPA3 receptor-knockout male animals did not develop the testis, and spermatogenesis (Choi et al., Biochemica Biophysica Acta 1781, 531-539, 2008; Ye X. Hum Reprod Update. 2008 14, 519-536, 2008).

On the basis of the fact that gintonin, even at a very small level, gives rise to an increase in intracellular free $Ca^{2+}$ level through the pathway G protein → PLC → $IP_3$ receptor → ER $Ca^{2+}$ reservoir → $Ca^{2+}$ release, the present inventors transfected and expressed genes of various known GRCRs and ligand-unknown orphan GRCRs, which are involved in the pathway G protein → PLC → $IP_3$ receotpr → ER $Ca^{2+}$ reservoir → $Ca^{2+}$ release, and examine gintonin activity to identify the receptor of the animal membrane GPCRs which is responsible for the transduction of gintonin activity. As a result, gintonin was found to function as a new, ginseng-derived biologically active ligand capable of activating LPA1(edg-2), LPA2(edg-4), LPA3(edg-7), LPA4 and LPA5 receptors among the EDG family receptors to induce the increase of intracellular free $Ca^{2+}$ through the pathway pertussis toxin-sensitive and -insensitive G proteins $\rightarrow$ PLC $\rightarrow$ IP$_3$ receptor $\rightarrow$ ER $Ca^{2+}$ reservoir $\rightarrow$ $Ca^{2+}$ release, which leads to the present invention.

## Disclosure

### Technical Problem

It is thus the primary object of the present invention to provide the novel use of the glycolipoprotein geintonin isolated from ginseng.

It is another object of the present invention to provide the use of gintonin as an agent for the prevention, amelioration and treatment of lysophosphatidic acid (LPA) receptor-related diseases.

### Technical Solution

In order to accomplish the above objects, the present invention provides the novel use of gintonin, a glycolipoprotein isolated and identified from the herbal plant ginseng, as a ligand of lysophosphatidic acid (LPA) receptors.

Also, the present invention provides a method for identifying interaction between the ginseng-derived glycolipoprotein gintonin and the LPA receptors.

Further, the present invention provides the use of the ginseng-derived glycolipoprotein gintonin in activating LPA receptors to effectively increase intracellular calcium levels and as an agent for preventing, ameliorating and treating a disease caused by a decrease in calcium-dependent biological activity and/or intracellular calcium level.

### Advantageous Effects

As described above, the ginseng-derived glycolipoprotein gintonin exhibits biological and pharmaceutical functions through interaction with lysophosphatidic acid (LPA) receptors.

Particularly, gintonin binds to LPA receptors which, in turn, induces a transient increase of intracellular free $Ca^{2+}$ through a series of signaling pathway processes in various organs of cardiovascular, nerve, endocrine, reproductive, and immune systems, so that it can be used in promoting various calcium-dependent biological and pharmaceutical activities (e.g., invigoration, immunopotentiation, virility enhancement, angiogenesis, anti-diabetic function, etc.) and in preventing, ameliorating and treating various diseases related to calcium deficiency.

Further, the composition of the present invention is effective for preventing, ameliorating and treating growth troubles attributed to calcium deficiency.

### Description of Drawings

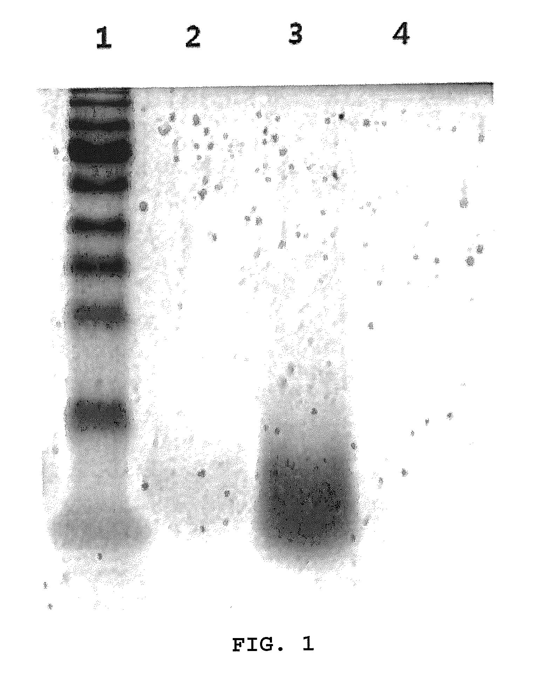

FIG. 1 shows the gintonin run on agarose by electrophoresis and visualized with Coomassie Brilliant Blue before and after dialysis (lane 1: molecular mark; lane 2: gintonin before methanol dialysis; lane 3: inner dialysate left after methanol dialysis; lane 4: outer dialysate after methanol dialysis).

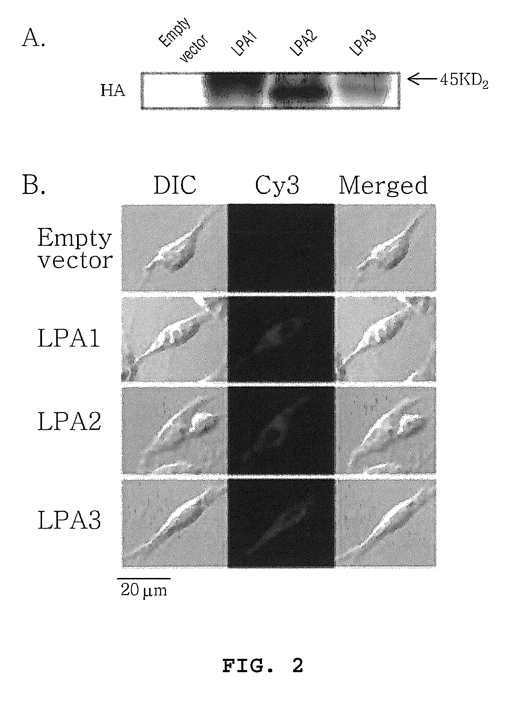

FIG. 2 shows the expression of LPA receptors on the membrane of B103 cells after transient transfection of en empty vector, a haematoglutin(HA)-tagged LPA1 receptor, an HA-tagged LPA2 receptor, and an HA-tagged LPA3 receptor by Western blotting using an anti-HA primary antibody and a second antibody (A) and by confocal laser microscopy (B) using an anti-HA primary antibody and a fluorescent Cy3-conjugated secondary antibody (B).

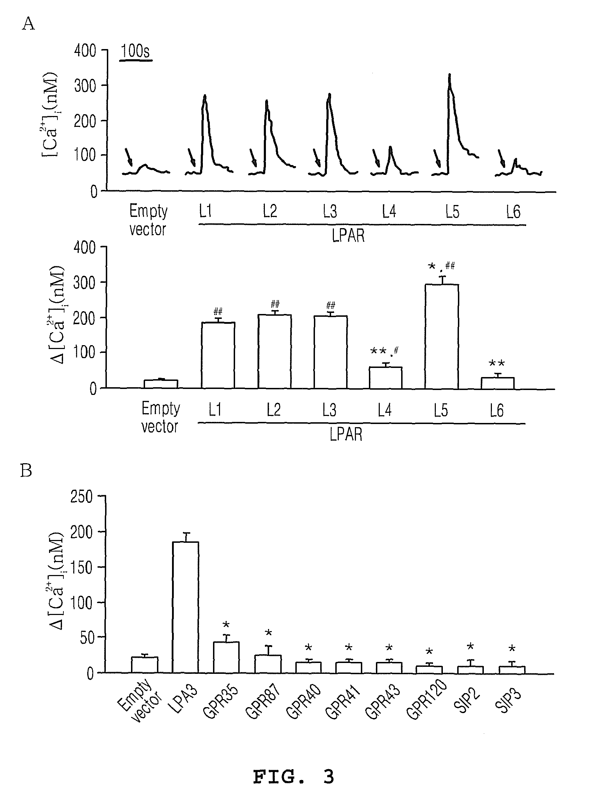

FIG. 3 shows the effect of gintonin (1 μg/ml) on intracellular calcium levels of B103 cells after transient transfection of an empty vector, an LPA1 receptor (L1), an LPA2 receptor (L2), an LPA3 receptor (L3), an LPA4 receptor (L4), an LPA5 receptor (L5), and an LPA6 receptor (L6) into the B103 cells ($^{\#}p<0.05$, $^{\#\#}p<0.001$, compared to empty vector; $^{*}p<0.05$, $^{**}p<0.001$, compared to LPA3 receptor) (A), and after transient transfection of GPR35, GPR87, both activated by LPA, GPR40, GPR41, GPR43, and GPR120, all known as fatty acid receptors, S1P1 and S1P2 receptors ($^{*}p<0.001$, LPA3 compared to receptor-expressing cells) (B).

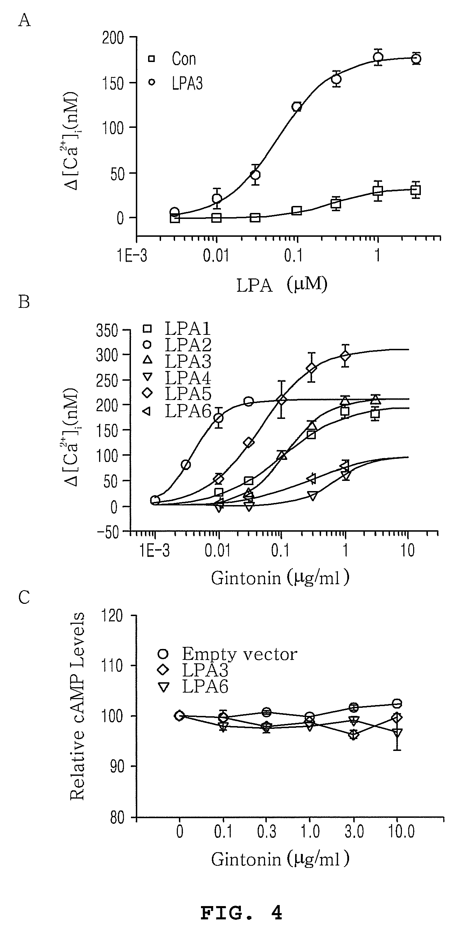

FIG. 4 shows changes in the intracellular calcium level of B103 cells with treatment with various concentrations of LPA after transient transfection of an empty vector an LPA3 receptor (A), and with treatment with various concentrations of gintonin after transient transfection of an empty vector, and LPA1, LPA2, LPA3, LPA4, LPA5 and LPA6 receptors (B), and changes in cAMP levels of B103 cells with various concentrations of gintonin after transient transfection of an empty vector, an LPA3 receptor, and an LPA6 receptor (C).

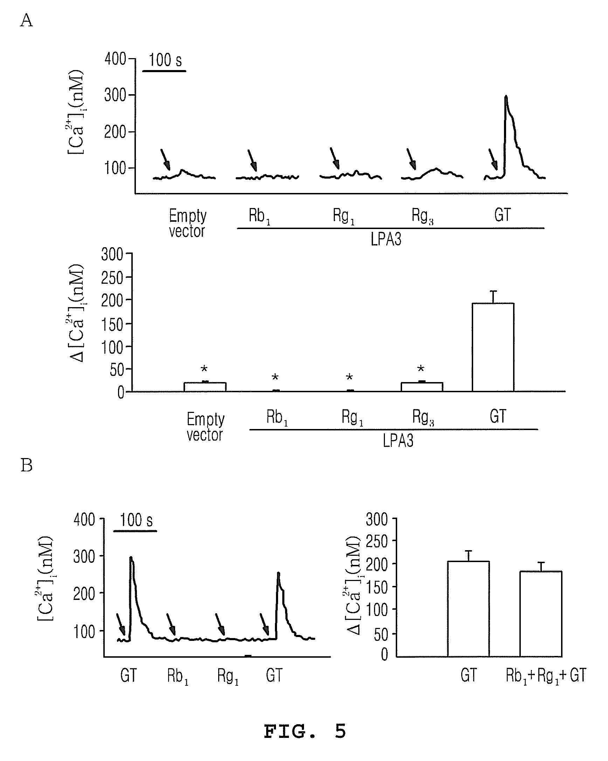

FIG. 5 shows changes in the intracellular calcium level of B103 cells with treatment with ginsenosides Rb1, Rg1 and Rg3 and gintonin after transient transfection of an empty vector and an LPA3 receptor into the B103 cells (*p<0.001, compared to gintonin (GT)-treated LPA3 receptor-expressing cells) (A), and with gintonin in the presence of ginsenoside Rb1 or Rg1 after transient transfection of an empty vector, or an LPA3 receptor into the B103 cells (B).

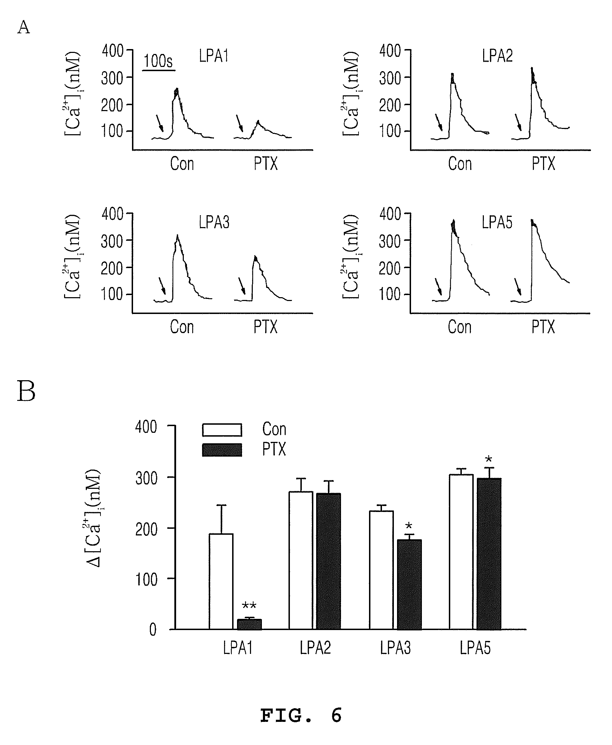

FIG. 6 shows gintonin (1 $\mu$g/ml)-induced changes of intracellular calcium levels in PTX-treated or PTX-non-treated B103 cells after transient transfection of LPA1, LPA2, LPA3 or LPA5 receptor into the B103 cells (A), and a histogram of results of (A) (*p<0.05, **p<0.001, compared to PTX-non-treated) (B).

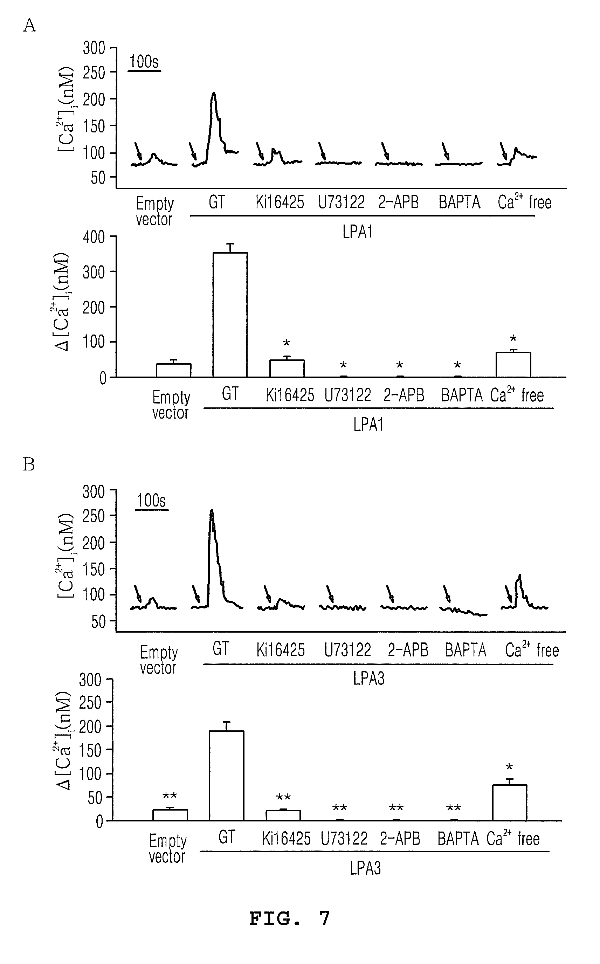

FIG. 7 shows changes in intracellular calcium levels of B103 cells with treatment with gintonin in the presence of an LPA receptor antagonist (Ki16425), a PLC inhibitor (U73122), an IP3 receptor antagonist (2-APB), or an intracellular calcium chelator (BAPTA) after the transient transfection of LPA1 receptor into the B103 cells and in a $Ca^{2+}$-free buffer (*p<0.001, compared to gintonin (GT) treated) (A), and after transient transfection of LPA3 receptor and in a $Ca^{2+}$-free buffer (*p<0.01, **p<0.001, compared to gintonin(GT)-treated, LPA3 receptor-expressing cells) (B).

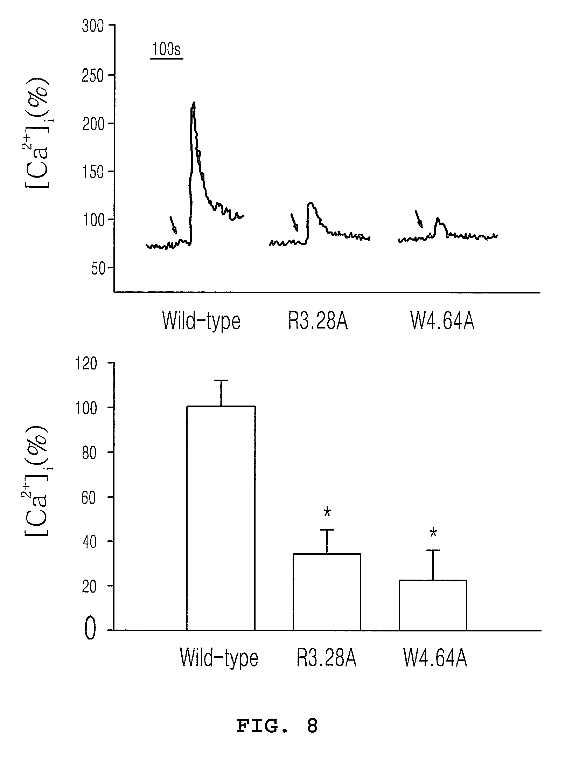

FIG. 8 shows changes in intracellular calcium levels of B103 cells with treatment with gintonin after transient transfection of a wild-type LPA3 receptor, a mutant R3.28A LPA3 receptor, or a mutant W4.64A LPA3 receptor into the B103 cells (*p<0.001, compared to wild-type LPA3 receptor).

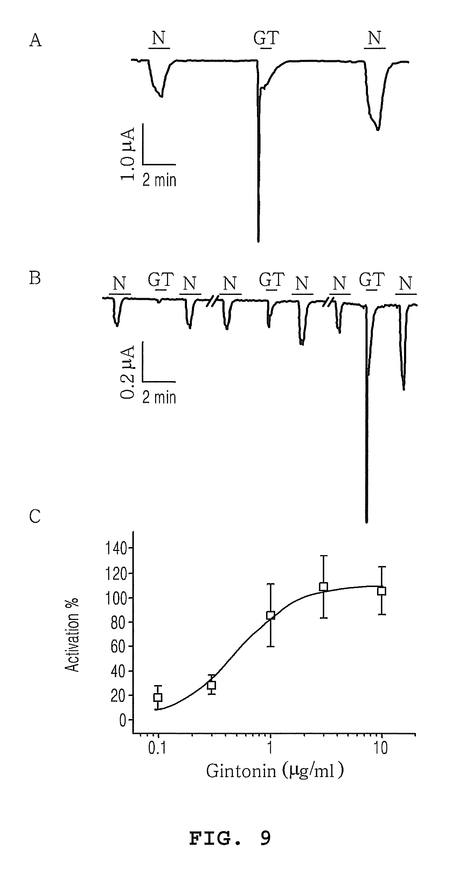

FIG. 9 shows gintonin-induced activation of NMDA receptors in *Xenopus* oocytes expressing NMDA receptors: In (A), an inward current in NMDA receptor-expressing *Xenopus* oocytes is induced by treatment with NMDA (300 $\mu$M). After depolarization, gintonin (1 $\mu$g/ml) activates LPA receptors endogenous to Xenopus oocytes, which, in turn, increases CaCC. Subsequent treatment with NMDA causes a larger inward current than before gintonin treatment, indicating that gintonin treatment induces NMDA receptor activation: (B) shows gintonin-induced NMDA receptor activation in a concentration-dependent manner: and (C) is a concentration-response curve illustrating gintonin-induced NMDA receptor activation.

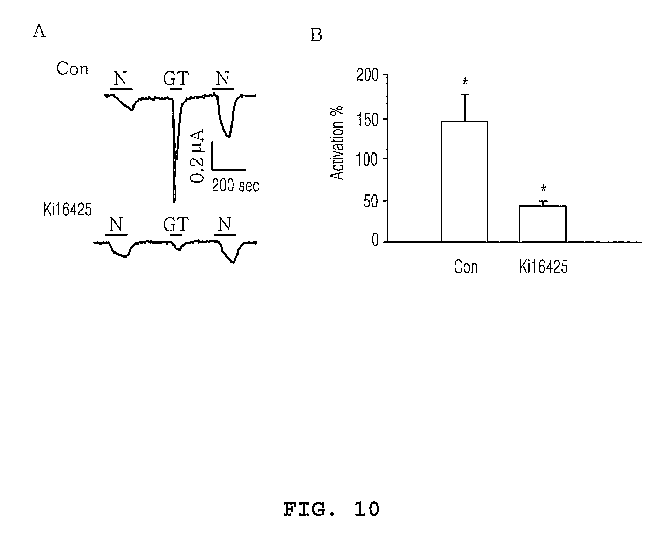

FIG. 10 shows the blocking activity of Ki16425, an antagonist of LPA1 and LPA3 receptors, against gintonin (1 $\mu$g/ml)-induced NMDA receptor activation in representative traces (A) and a histogram (B) (*p < 0.01, compared to the control (Con)).

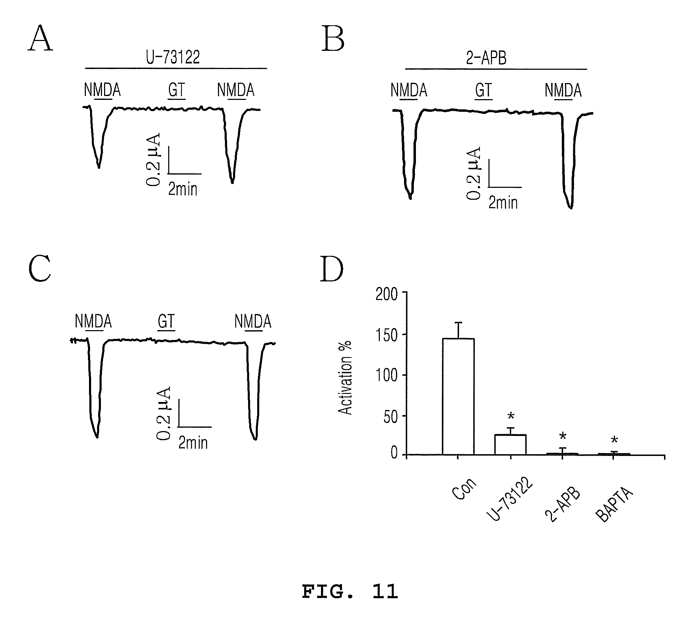

FIG. 11 shows membrane signaling pathways upstream of gintonin (1 $\mu$g/ml)-induced NMDA receptor activation by illustrating the inhibitory activity of the active PLC inhibitor U73122 (1 $\mu$M) (A), the $IP_3$ receptor antagonist 2-APB(100 $\mu$M) (B), and $Ca^{2+}$ chelator BAPTA (100 $\mu$M, 3 h treated in *Xenopus* oocytes) (C) against gintonin-induced activation of endogenous CaCC and NMDA receptors, and in a histogram summarizing the inhibitory activities of U-73122, 2-APB, and BAPTA (*p < 0.01, compared to control (Con)).

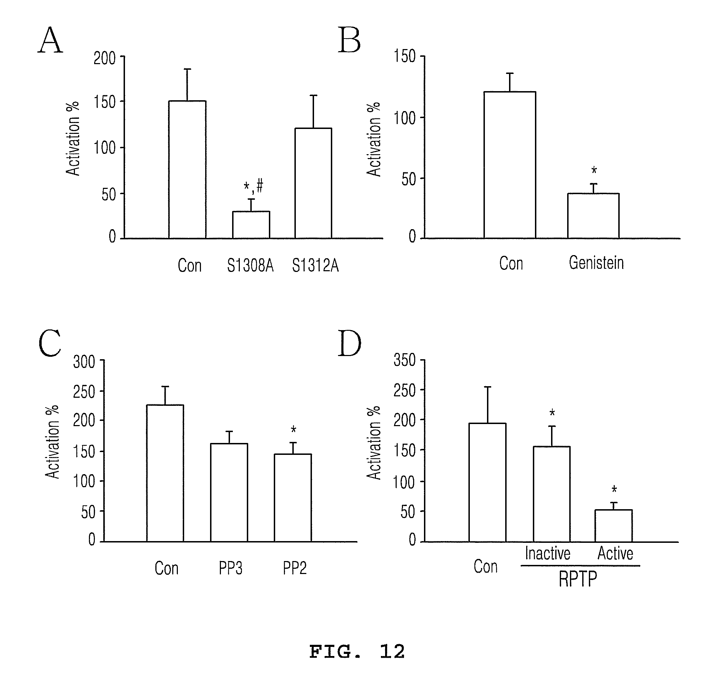

FIG. 12 shows signaling pathways downstream of gintonin-induced NMDA receptor activation by illustrating a low level of gintonin-induced NMDA receptor activation in cells expressing a mutant NMDA receptor (S1308A) in which the serine at position 1308 (S1308), known as a PKC phosphorylation site, was substituted by alanine (*p < 0.01, compared to control, #p < 0.01, compared to oocytess expressing mutant S1312A receptor) (A), the inhibitory activity of genistein (10 $\mu$M), known as a tyrosine kinase inhibitor, against gintonin-induced NMDA receptor activation (*p < 0.01, compared to the control) (B), the inhibitory activity of PP2, known as an active inhibitor of Src-family kinase, against gintonin-induced NMDA activation and the non-inhibitory activity of PP3 (30 $\mu$M), known as an inactive inhibitor, against gintonin-induced NMDA activation (C), and a low level of NMDA receptor activity in oocytes expressing active or inactive phosphoprotein phosphatase (RPTP) $\alpha$ when treated with gintonin (D).

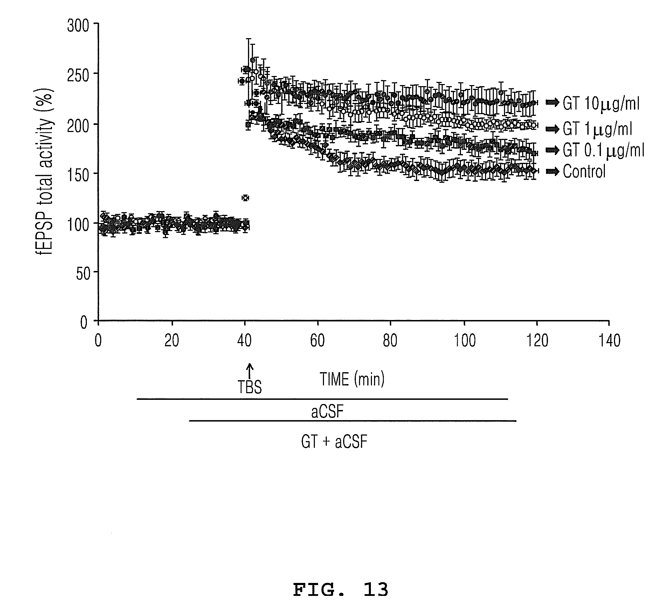

FIG. 13 shows gintonin-induced long-term potentiation in hippocampal slices (red arrow: time of triggering theta burst stimulation (TBS)).

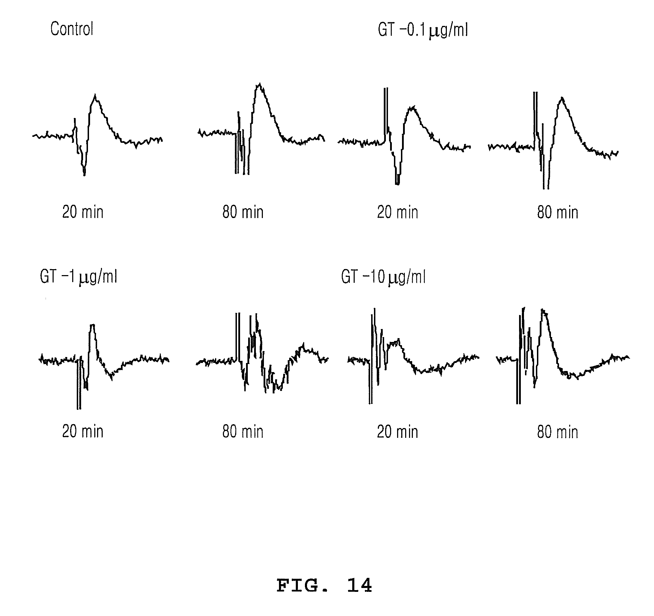

FIG. 14 shows representative time field traces of gintonin-induced LTP in rat hippocampus between the control and gintonin-treated groups (20 min) before TBS and 80 min after TBS

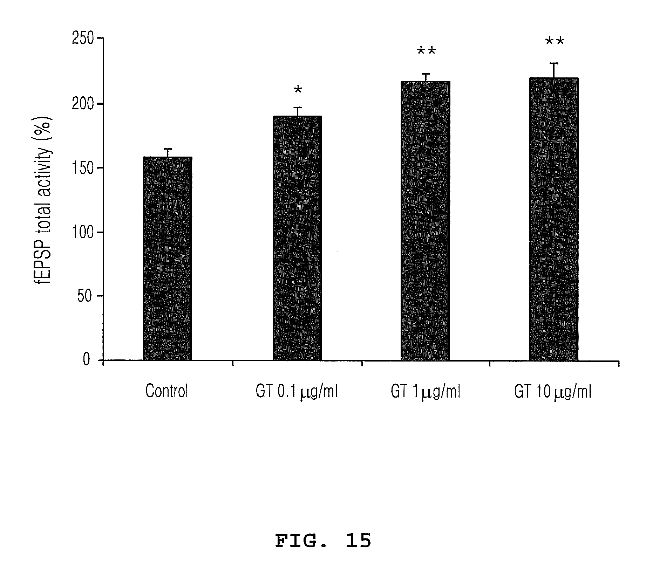

FIG. 15 is a histogram showing an increase of gintonin-induced rat hippocampal LTP in a concentration-dependent manner (*p < 0.05, **p < 0.01, compared to the control).

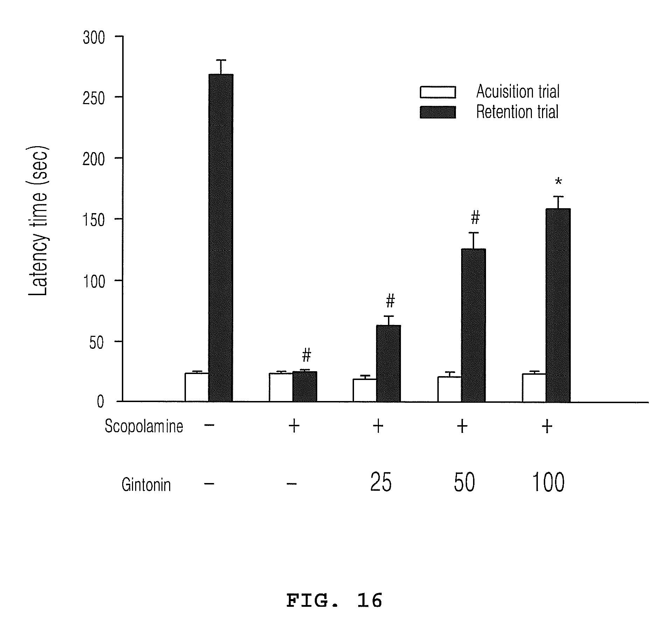

FIG. 16 shows the protective activity of gintonin against scopolamine-caused long-term memory impairment in a dose-dependent manner, as analyzed by a passive avoidance test (#p < 0.0001, group not treated with scopolamine; *p < 0.01, group not administered with gintonin; tacrine for positive control, data not shown)

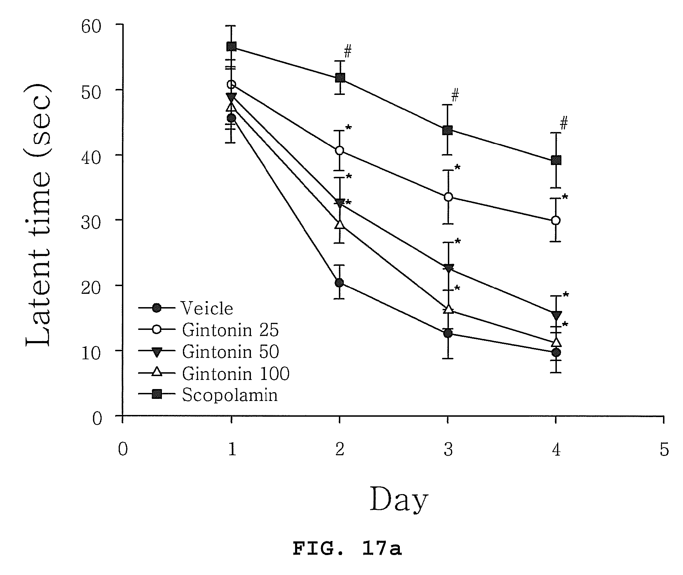

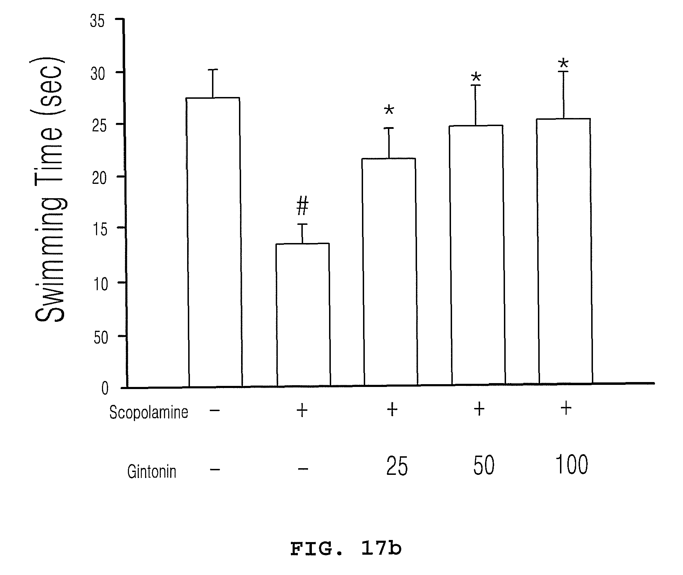

FIG. 17 shows the protective activity of gintonin against spatial cognition and simple spatial memory impairment, as analyzed by a Morris water maze test, FIG. 17a shows by illustrating an improvement effect on scopolamine-caused spatial cognition impairment (*p < 0.01, compared to scopolamine-administered group, day; #p <0.001, compared to group administered with scopolamine alone; tacrine group for positive control, data not shown) and FIG. 17b shows by depicting swimming time during which the mice swam in the quadrant where the rescue platform had been located.

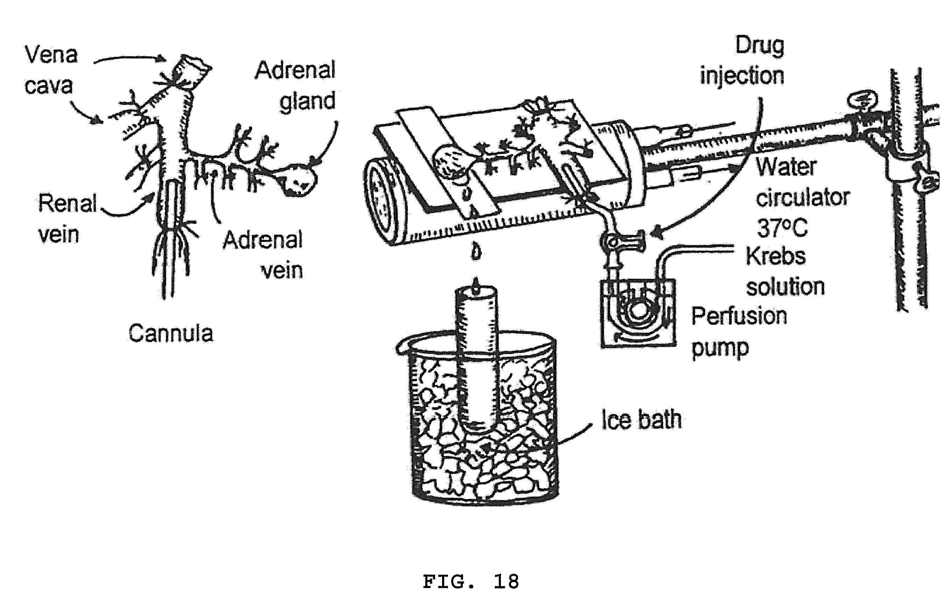

FIG. 18 is diagram illustrating an experiment for catecholamine secretion from the adrenal gland isolated from a rat.

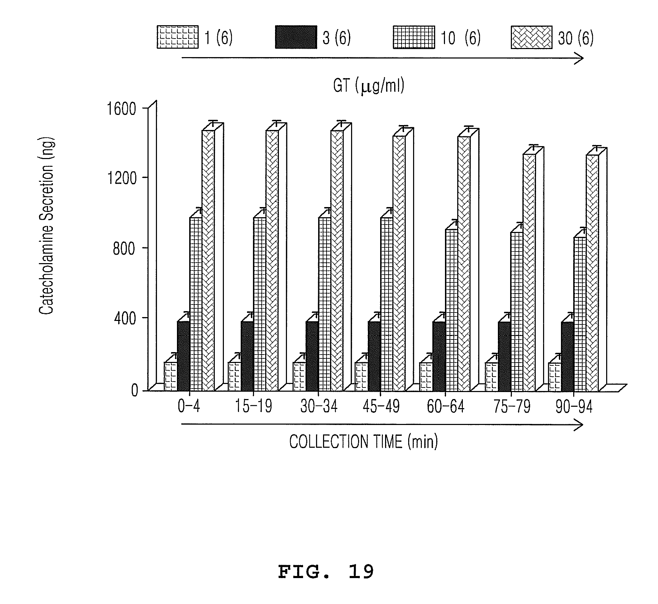

FIG. 19 shows gintonin-induced catecholamine secretion as analyzed using perfusates collected for 4 min at 15 min intervals after treatment with different concentrations of gintonin (numerals in parentheses represent numbers of used adrenal glands, X-axis represents amounts of catecholamine secreted for 4 min (ng/perfusate), and Y-axis represents time to collect the perfusate).

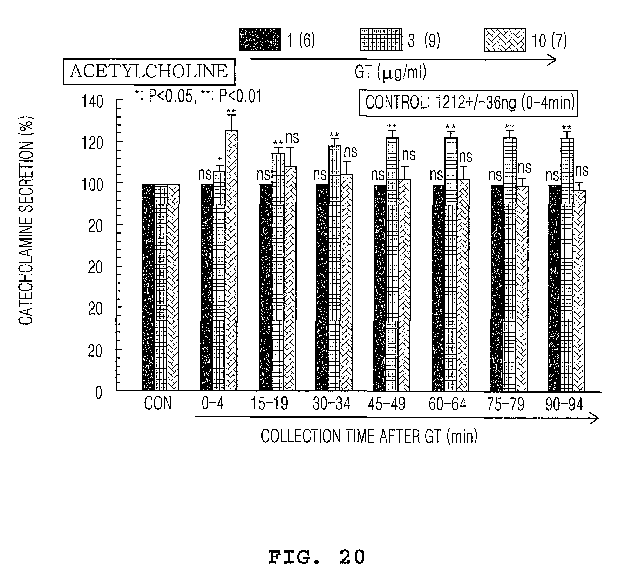

FIG. 20 shows effect of gintonin on acetylcholine-induced secreation of catecholamine after various concentrations of gintonin are applied in the presence of acetylcholine (5.32 mM) (*p < 0.05, **p < 0.01, ns: statistically insignificant).

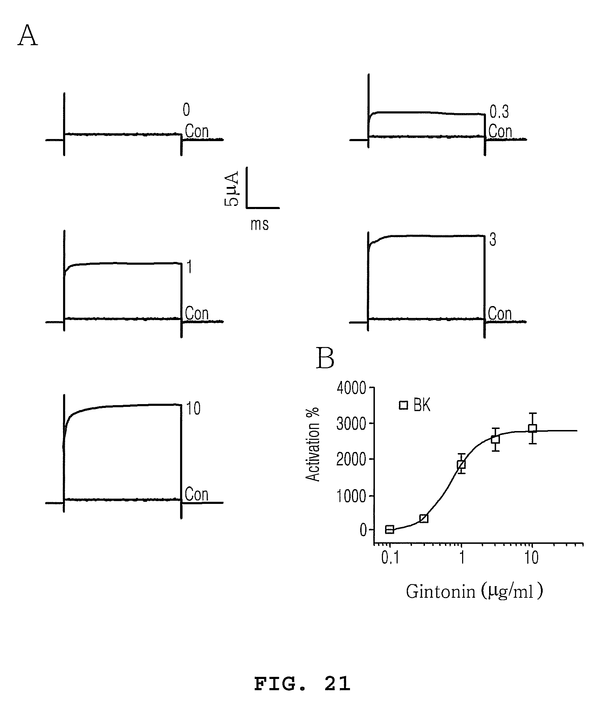

FIG. 21(A) shows gintonin-induced outward currents of the $BK_{Ca}$ $K^+$ channels expressed in *Xenopus* oocytes according to concentrations of gintonin, and (B) is a concentration-response curve illustrating gintonin-induced currents of $BK_{Ca}$ $K^+$ channels.

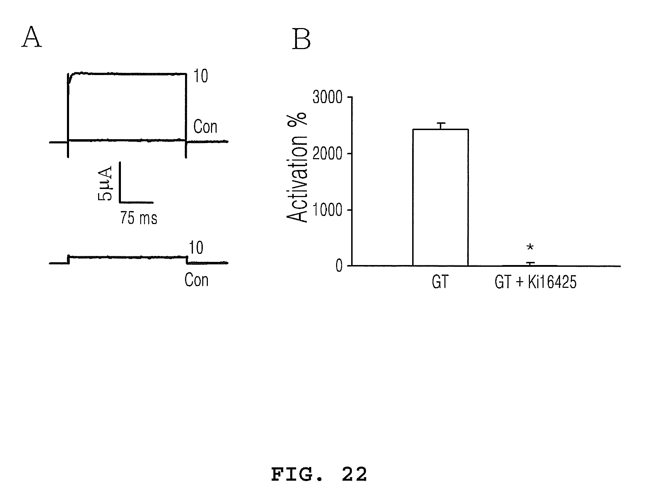

FIG. 22 shows the blocking activity of Ki16425, an antagonist of LPA1 and LPA3 receptors, against gintonin-induced $BK_{Ca}$ $K^+$ channel activation in representative traces recorded at 10 $\mu$M of Ki16425 (A) and in a histogram (*p < 0.01, compared to the control (Con)) (B).

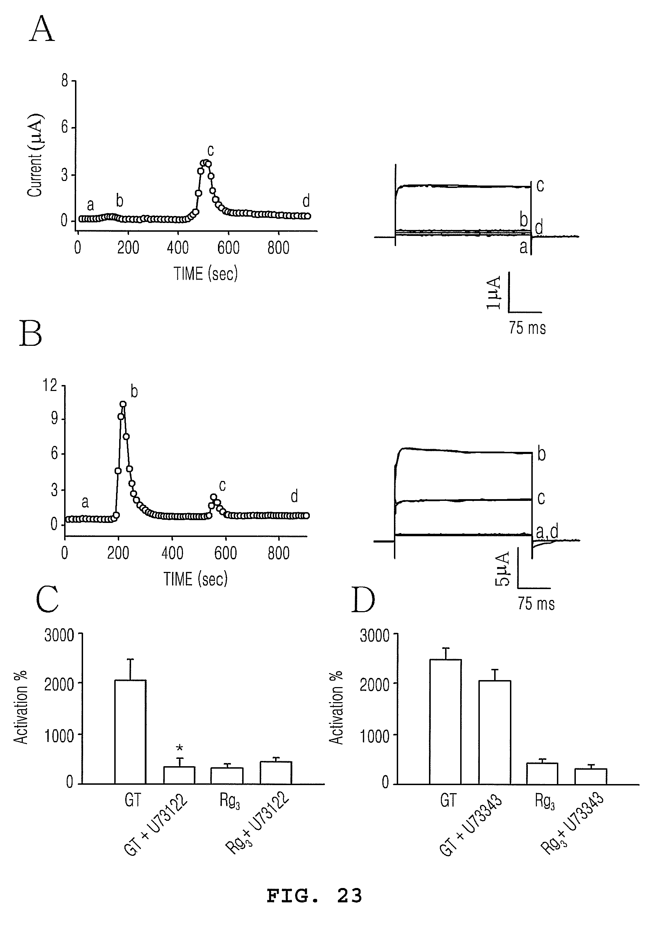

FIG. 23 shows effects of active and inactive PLC inhibitors on gintonin- and ginsenoside Rg3-induced $BK_{Ca}$ $K^+$ channel activation by illustrating $BK_{Ca}$ $K^+$ channel activity upon treatment with gintonin or ginesnoside Rg3 in the presence of active PLC inhibitor U73122(1 $\mu$M) in a time-current relationship (left) and representative traces (right) (A), $BK_{Ca}$ $K^+$ channel activity upon treatment with gintonin or ginesnoside Rg3 in the presence of inactive PLC inhibitor U73443 in a time-current relationship (left) and representative traces (right) (B), the blocking activity of U73122 (1 $\mu$M) against gintonin-induced $BK_{Ca}$ $K^+$ channel activation in a histogram (*p < 0.01, compared to the control (Con)) (C), and the blocking activity of U73343 (1 $\mu$M) against gintonin-induced $BK_{Ca}$ $K^+$ channel activation in a histogram (D) (a. prior to gintonin treatment; b. gintonin treatment; c. ginsenoside $Rg_3$ treatment; d. drug washing out).

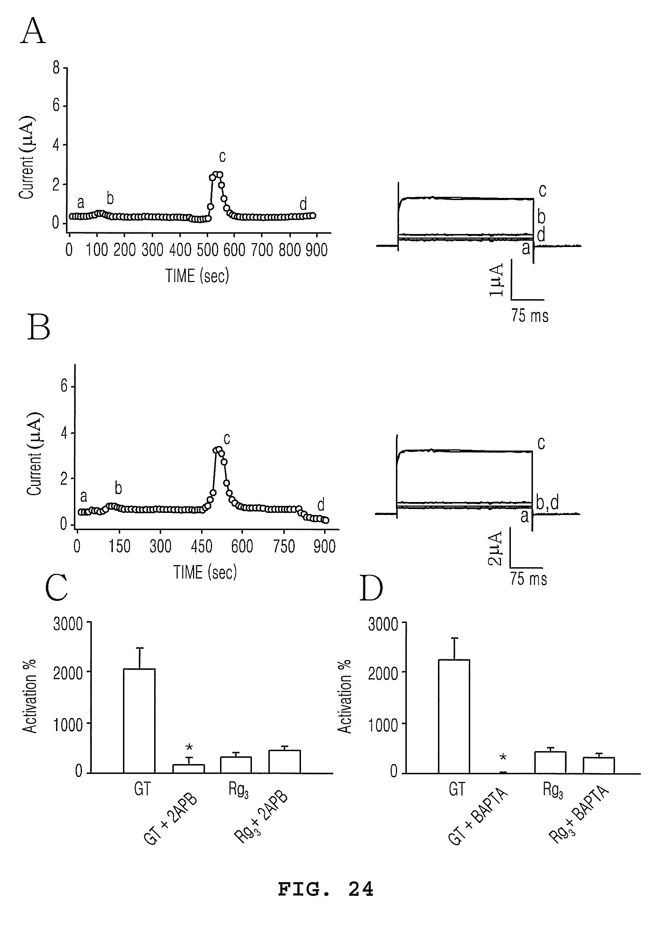

FIG. 24 shows effects of an $IP_3$ receptor antagonist and a calcium chelator on gintonin- and ginsenoside Rg3-induced $BK_{Ca}$ $K^+$ channel activation by illustrating $BK_{Ca}$ $K^+$ channel activity upon treatment with gintonin or ginesnoside Rg3 in the presence of the $IP_3$ receptor antagonist 2-APB (100 $\mu$M) in a time-current relationship (left) and representative traces (right) (A), $BK_{Ca}$ $K^+$ channel activity upon treatment with gintonin or ginesnoside Rg3 in the presence of the calcium chelator BAPTA-AM in a time-current relationship (left) and representative traces (right) (B), the blocking activity of 2-APB (100 $\mu$M) against gintonin-induced $BK_{Ca}$ $K^+$ channel activation in a histogram (*p < 0.01, compared to the control(Con)) (C), and the blocking activity of BAPTA (100 $\mu$M, 3h) against gintonin-induced $BK_{Ca}$ $K^+$ channel activation in a histogram (D) (a. prior to gintonin treatment; b. gintonin treatment; c. ginsenoside $Rg_3$ treatment; d. drug washing out).

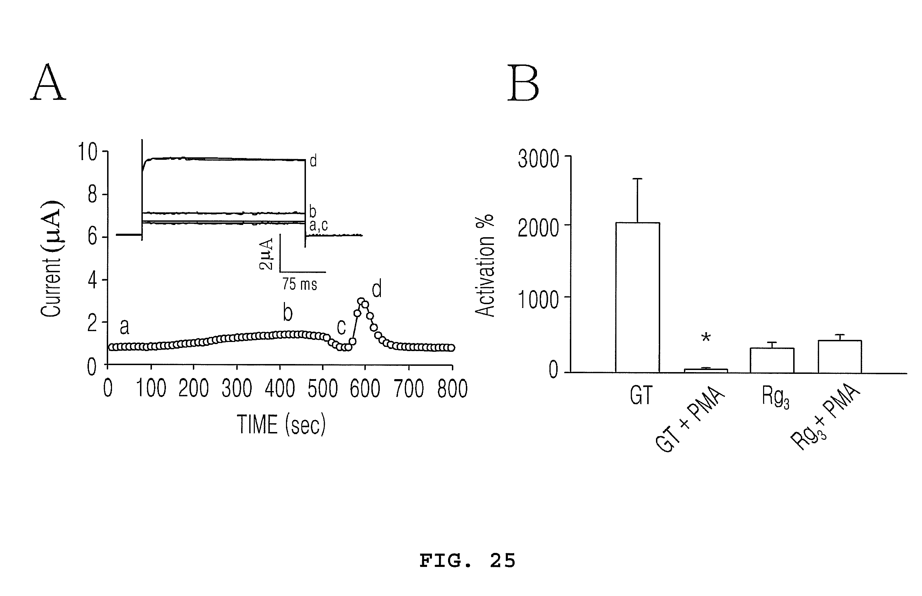

FIG. 25 shows the effect of a PKC activator on gintonin- and ginsenoside Rg3-induced $BK_{Ca}$ $K^+$ channel activation by illustrating $BK_{Ca}$ $K^+$ channel activity upon treatment with gintonin or ginesnoside Rg3 in the presence of PMA(1 $\mu$M) in a time-current relationship (lower) and representative traces (upper) (A), and the blocking activity of PMA(100 nM, 3h) against gintonin-induced $BK_{Ca}$ $K^+$ channel activation in a histogram (B) (a. prior to gintonin treatment; b. gintonin treatment; c. ginsenoside $Rg_3$ treatment; d. before ginsenoside treatment).

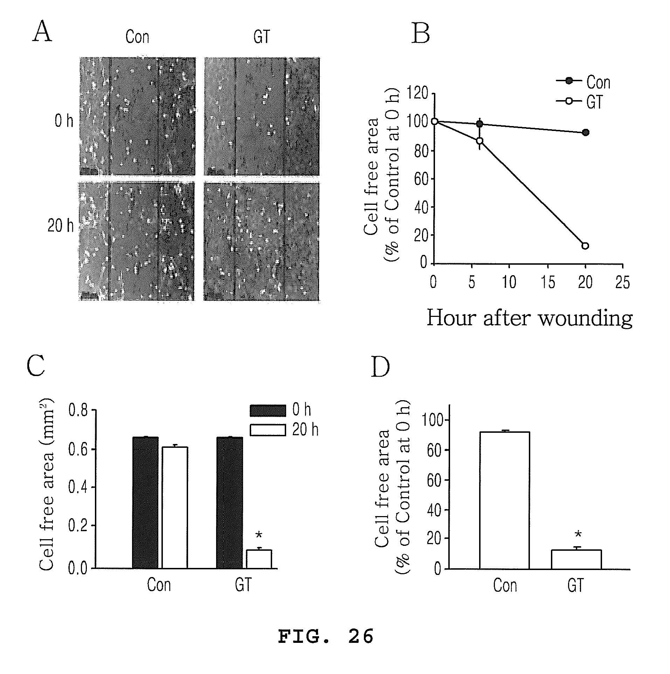

FIG. 26 shows the effect of gintonin on wound healing: In (A), after scratch wounds were made by dragging a pipette tip across monolayers of HUVECs, incubation with gintonin (30 $\mu$g/ml) for 20 hrs covered the wound area while the cell-free paths were not covered with cells in the control group: (B) shows the relationship of wound healing in a gintonin-treated group and a non-treated group (control) with time: (C) shows % cell-free areas of the group treated with gintonin (30 $\mu$g/ml) compared to the control (*p <0.01, compared to the control, VG: vascular endothelial growth factor (VEGF)).

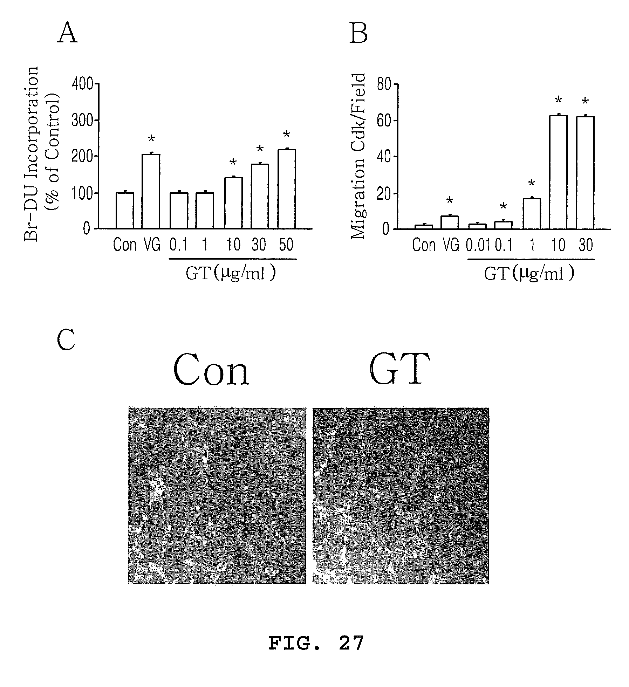

FIG. 27 shows the effect of gintonin on tubular formation by identifying that gintonin promotes the incorporation of Br-DU into DNA of HUVECs in a dose-dependent manner (*p <0.01, compared to the control) (A), the migration of HUVECs in a dose-dependent manner (*p <0.01, compared to the control) (B), and angiogenesis resulting from HUVEC proliferation and migration (C).

## Best Mode

Below, a detailed description will be given of the present invention.

The present invention addresses provides the use of gintonin, a glycolipoprotein isolated and identified from ginseng, as a ligand of lysophosphatidic acid (LPA) receptor subtypes.

Particularly, the present invention provides a novel ligand binding to an LPA receptor.

The novel ligand according to the present invention is the ginseng-derived glycolipoprotein gintonin having a structural protein composed of ginseng major latex-like protein (MLP151) and ribonuclease-like storage proteins.

The ginseng major latex-like protein (MLP151) may comprise amino acid sequences set forth as SEQ ID NOS: 1 to 4. In addition, the MLP151 may have an amino acid sequence set forth as SEQ ID NO: 5, with three N-glycosylation sites.

SEQ ID NO: 5:

mgltgklicq tgiksdgdvf helfgtrphh vpnitpaniq gcdlhegefg kvgsvviwny
sidgnamiak <u>eeivaideed ks</u>vtfkvveg hlfeefksiv fsvhvdtkge dnlvtwsidy
<u>ekl *nes* vkdp tsyldfllsv trdieahhlp k</u>
wherein, the underlined regions correspond to peptide fragments (SEQ ID NOS: 1~4) established by proteomics analysis, and italicized amino acid regions represent N-glycosylation sites.

The ginseng ribonuclease(RNAse)-like major storage protein may have the amino acid sequence set forth as SEQ ID NO: 11 containing the amino acid sequences of SEQ ID NOS: 6 to 10.

SEQ ID NO: 11:

mraiyiisvi ivslsifswg gna<u>rsdypwa m</u>falrlqwpa gfcevnnacd <u>tksllntfti</u>
<u>hglypynakg</u> tpalycdgta fdvnsvsdfl aemhlawpsh etntediqfw ehewkkhgrc
seallkqtdy <u>frtalafrka fdivgllnqe giypnndlyr pkmikeaikk hlnavpeidf</u>
<u>tknenseyvl</u> tdinvcvnqq atrfvdcptd datddyrlkf vrlpskmkfa dprtnsii
wherein the underlined regions corresponds to peptide fragments (SEQ ID NOS: 6~10) established by protemics analysis.

In one embodiment of the present invention, the gintonin acts as an agonist of LPA receptors.

In another embodiment, the gintonin may be sourced from roots, stems, and leaves of various ginsengs including fresh ginseng, white ginseng, red ginseng, artificially sown but wild-grown ginseng, artificially bred ginseng, and wild ginseng. American ginseng and Chinese ginseng as well as Korean ginseng are used in the present invention. Preferably and in a nonlimiting fashion, Korean red ginseng (*Panax ginseng* C. A. Meyer) grown for four to six years is used.

Also, contemplated in accordance with another aspect of the present invention is a method for identifying interaction between gintonin and an LPA receptor.

LPA family receptors are endogenous to cells of most organs. B103 rat neuroblastoma cells, known as LPA receptor-null cells (Valentine et al., Biochim Biophys Acta 1780. 597-605) were transiently transfected with a plasmid (vector) carrying one of genes coding for LPA1(edg-2), LPA2(edg-4), LPA3(edg-7), LPA4, LPA5 and LPA6 receptors or a plasmid carrying none of them (empty vector). Two or three days after transfection, the cells are suspended, and made permeable. Then, the permeable cells are treated with Fura-2AM, a fluorescent dye binding to calcium, and then with gintonin, followed by spectrofluorephotometry to examine whether gintonin induces an increase in intracellular free $Ca^{2+}$ level.

In detail, the method for identifying interaction between gintonin and an LPA receptor comprises:

(1) preparing large quantities of plasmids carrying respective LPA family receptor genes and an empty plasmid carrying none of them through amplification and purification (maxi-preparation); (2) verifying the expression of LPA receptors wherein B103 cells are transfected with haematoglutin (HA)-tagged LPA receptors, and then subjected to Western blotting analysis using an anti-HA primary antibody and a horseradish peroxidase (HRP)-conjugated secondary antibody to develop a color; (3) verifying the expression of LPA receptors wherein B1-3 cells are transfected with haematoglutin (HA)-tagged LPA receptors, and then subjected to confocal laser microscopy using an anti-HA antibody and a fluorescence dye Cy3-conjugated secondary antibody; (4) transfecting the empty plasmid and each of the plasmids carrying LPA family receptor genes into B103 cells; (5) treating the transfected B103 cells with trypsin (0.05% trypsin with EDTA, w/v) 2~3 days post-transfection, to give a cell suspension; (6) culturing the suspended B103 cells with Fura-2AM (2.5 $\mu$M); and (7) treating the suspended B103 cells with gintonin and quantifying a change in intracellular free $Ca^{2+}$ level in a cuvette by spectrofluorephotometry using Fura-2AM.

Optionally, the method may further comprise (8) pretreating the suspended B103 cells with LPA receptor antagonists and LPA receptor-mediated signaling-relevant drugs (e.g., pertussis toxin, PLC inhibitors, IP$_3$ receptor antagonists) to examine whether the cells decrease or increase in intracellular free $Ca^{2+}$ prior to treatment with gintonin; (9) performing site-directed mutagenesis to identify an amino acid of LPA receptors with which gintonin interact to activate the LPA; and (10) examining whether gintonin activates orphan GPCRs including GPR35 and GPR87, and free fatty acid GPCRs including GPR40, GPR41, GPR43 and GPR120, all known for activation by LPA.

In accordance with a further aspect thereof, the present invention provides the use of gintonin in activating LPA receptors to effectively increase intracellular calcium levels, and as an agent for preventing, ameliorating and treating a disease caused by a decrease in calcium-dependent biological activity and/or intracellular calcium level.

According to one embodiment, the present invention provides a pharmaceutical composition for improving learning ability and memory by NMDA receptor activation and hippocampal LTP enhancement, comprising the ginseng-derived glycolipoprotein gintonin or a pharmaceutically acceptable salt thereof as an active ingredient.

In another embodiment, the present invention provides a pharmaceutical composition for increasing resistance to stress and recovery from stress-induced fatigue, comprising the ginseng-derived glycolipoprotein gintonin or a pharmaceutically acceptable salt thereof as an active ingredient.

In another embodiment, the present invention provides a pharmaceutical composition for wound healing, comprising the ginseng-derived glycolipoprotein gintonin or a pharmaceutically acceptable salt thereof as an active ingredient.

In another embodiment, the present invention provides a pharmaceutical composition for the prevention and treatment of a disease associated with vascular smooth muscle proliferation, comprising the ginseng-derived glycolipoprotein gintonin or a pharmaceutically acceptable salt thereof as an active ingredient.

In another embodiment, the present invention provides a pharmaceutical composition for the prevention and treatment of inflammation, comprising the ginseng-derived glycolipoprotein gintonin or a pharmaceutically acceptable salt thereof as an active ingredient.

In another embodiment, the present invention provides a pharmaceutical composition for the prevention and treatment of a calcium deficiency-associated disease, comprising the ginseng-derived glycolipoprotein gintonin or a pharmaceutically acceptable salt thereof as an active ingredient.

In another embodiment, the present invention provides a pharmaceutical composition for the prevention and treatment of a neurodegenerative disease caused by neural death, comprising the ginseng-derived glycolipoprotein gintonin or a pharmaceutically acceptable salt thereof as an active ingredient.

As used herein, the calcium-dependent biological activity means may be relevant to invigoration, immunopotentiation, virility enhancement, angiogenesis, or anti-diabetic function. Examples of the disease associated with vascular smooth muscle proliferation include postoperative stenosis and recurrent stenosis. The calcium deficiency-associated disease may be selected from the group consisting of schizophrenia, Alzheimer's disease, Hungtington's disease, familial hemiplegic migraine, epilepsy, episodic ataxia, and spinocerebellar ataxias. Examples of the neural death-caused neurodegenerative disease include, but are not limited to, stroke, cerebral palsy, memory impairment, dementia, amnesia, Parkinson's disease, Pick disease, and Creutzfeld-Jakob disease.

As ginseng has long been used as a source of herb medicines with safety, the gintonin of the present invention, isolated from ginseng, can be safely used without toxicity and side effects.

The pharmaceutical composition for the prevention and treatment of a low calcium-dependent biological activity- and/or calcium deficiency-associated disease in accordance with the present invention comprises gintonin in an amount of from 0.0001 to 10 wt%, preferably in an amount of from 0.001 to 1 wt%, based on the total weight thereof.

In addition, the composition of the present invention may further comprise suitable carriers, excipients or diluents typically used in the pharmaceutical art. The dosage of gintonin of the present invention may be used alone or in combination with other pharmaceutically active compounds as well as in an appropriate assembly.

The pharmaceutical composition comprising gintonin in accordance with the present invention may be formulated into oral preparations such as powders, granules, tablets, capsules, suspensions, emulsions, syrups, aerosols, etc., or parenteral preparations such as external applications, suppositories and sterile injections. Among the carriers, diluents or excipients useful in the pharmaceutical composition are lactose, dextrose, sucrose, sorbitol, mannitol, xylitol, erythritol, maltitol, starch, acacia gum, aginate, gelatin, calcium phosphate, calcium silicate, cellulose, methyl cellulose, crystalline cellulose, polyvinyl pyrrolidone, water, methylhydroxybenzoate, propylhydroxybenzoate, talc, magnesium stearate and mineral oil. The active ingredient may be formulated in combination with a diluents or excipients such as a filler, a thickener, a binder, a humectant, a disintegrant, and a surfactant. Solid preparations intended for oral administration may take the form of tablets, pills, powders, granules, capsules, and the like. In regard to these solid agents, the active ingredient in the present invention is formulated in combination with at least one excipient such as starch, calcium carbonate, sucrose, lactose, or gelatin. In addition to such simple excipients, lubricants such as magnesium stearate and talc may be used. Liquid preparations intended for oral administration include suspensions, internal use solutions, emulsion, syrups, and the like. In addition to a simple diluent such as water or liquid paraffin, various excipients, such as wetting agents, sweetening agents, aromatics, preservatives, and the like may be contained in the liquid preparations. Also, the pharmaceutical composition of the present invention may be administered via a non-oral route. For this, sterile aqueous solutions, non-aqueous solvents, suspensions, emulsions, lyophilizates, suppositories, and the like may be used. Injectable propylene glycol, polyethylene glycol, vegetable oils such as olive oil, and esters such as ethyl oleate, may be suitable for non-aqueous solvents and suspensions. The basic materials of suppositories include Witepsol, macrogol, Tween 61, cacao butter, laurin butter, and glycerogelatin.

The dosage of the composition of the present invention may vary depending on various factors including the patient's condition and weight, the severity of disease, dosage form, the route of administration and the time of administration, and can be suitably determined by the attending physician. To achieve the desired effects, however, the composition of the present invention may be preferably administered at a daily dose of from 0.0001 to 100 mg/kg. The composition may be administered in a single dose per day or in multiple doses per day. The dosage is not intended to limit the present invention in any way.

The pharmaceutical composition of the present invention may be administered to mammals such as rats, mice, livestock, and humans, via various routes. All routes of administration may be expected, for example, oral or intrarectal administration or intravenous, intramuscular, subcutaneous, intradural, or intracerebroventricular injection may be contemplated.

The composition comprising gintonin in accordance with the present invention may be applied to medicaments, foods and beverages for use in the prevention and treatment of calcium deficiency-associated diseases. For example, the gintonin may be added to various foods, beverages, gums, teas, vitamin complexes, health functional foods, etc.

Being almost free of toxicity and side effects, the gintonin of the present invention can be safely ingested for a long period of time for preventive purposes.

When added to foods or beverages to prevent diseases associated with low calcium-dependent biological activity and/or calcium deficiency, the amount of the gintonin used may be from 0.01 to 15 wt% based on the total weight of the food or beverage. For a health beverage, the gintonin of the present invention may be added in an amount of from 0.02 to 5 g per 100 ml and preferably in an amount of from 0.3 to 1 g per 100 ml.

No particular limitations are imparted to the other components of the health beverage composition so long as the gintonin of the present invention is used in an amount such as the one described above. Like conventional beverages, the health beverage of the present invention may further comprise various flavor modifiers or natural carbohydrates. Examples of the natural carbohydrates useful in the present invention include monosaccharides such as glucose, fructose, etc.; disaccharides such as maltose, sucrose, etc.; polysaccharides such as dextrin, cyclodextrin, etc.; and sugar alcohols such as xylitol, sorbitol, erythritol, etc. As for the flavor modifiers, they are advantageously natural flavor modifiers (taumatin, stebia extracts, i,e, Rebaudioside A, glycyrrhizin), and synthetic sweeteners (saccharin, aspartame, etc.). The amount of the natural carbohydrates used may be from 1 to 20 g and preferably from 5 to 12 g per 100 ml of the health beverage composition of the present invention.

In addition, the composition of the present invention may be supplemented with a variety of agents including nutrients, vitamins, minerals (electrolytes), flavoring agents, synthetic and/or natural, colorants, thickeners (cheese, chocolate), pectic acid or salts thereof, alginic acid or salts thereof, organic acids, protective colloidal thickening agents, pH modifiers, stabilizers, preservatives, glycerin, alcohols, carbonating agents used in carbonated beverages, etc. For use in the preparation of fruit or vegetable juices, the composition of the present invention may further comprise fresh fruit and/or vegetable soup. These components may be used separately or in combination. As a rule, the amount of the agents ranges from zero to 20 parts by weight per 100 weight parts of the composition.

A better understanding of the present invention may be obtained through the following examples which are set forth to illustrate, but are not to be construed as limiting the present invention.

EXAMPLE 1: Sequencing of Gintonin

1-(1). Methanol dialysis of gintonin

After being isolated from ginseng (refer to Korean Patent Application No. 10-2010-0052913), 200 mg of gintonin was dissolved in 10-15 ml of 100% (w/v), r and this solution was placed in a dialysis bag (pore size: 6,000~8,000, Sigma-Aldrich, St. Louis, USA), followed by dialysis against 30~50 volumes of 100% methanol for 48 hrs.

During dialysis, methanol was changed twice with a fresh one. After completion of dialysis, the inner dialysate, which remained within the dialysis bag, and the outer dialysate, which escaped from the dialysis bag, were dried, and quantitated in separation.

1-(2). *In-gel* tryptic digestion

The inner dialysate was dried, and 100 $\mu$g/ml of the dried inner dialysate was subjected to 10% SDS-PAGE.

FIG. 1 shows the gintonin run on agarose by electrophoresis and visualized with Coomassie Brilliant Blue before and after dialysis.

The gintonin band was broad with low intensity before dialysis against methanol (lane 2, FIG. 1). The inner dialysate left after methanol dialysis was detected as a broad band without a change in molecular weight, but with much greater intensity (lane 3, FIG. 1).

On the other hand, the outer dialysate was not stained with Coomassie Brilliant Blue (land 4, FIG. 1).

These results indicate that the lipid component of gintonin has an influence on the Coomassie Brilliant Blue staining of the protein component.

The protein stained with Coomassie blue was excised from the gel, and washed with a solution containing 25 mM ammonium bicarbonate and 50% (w/v) acetonitrile. The gel piece was incubated at 37°C for 18~20 hrs in 40 mM ammonium bicarbonate, 10% (w/v) acetonitrile, and 25 $\mu$g/ml trypsin (Promega, Madison, WI, USA).

After the tryptic digestion, the peptidyl digest was extracted twice with 50 mM ammonium bicarbonate, 50% acetonitrile and 5% (w/v) trifluoroacetic acid (TFA). The peptide extracts were pooled, lyophilized in a vacuum centrifuge, and stored at 4°C before use.

### 1-(3). Ion motility-mass spectrometry (IM-MS)

After *in-gel* tryptic digestion, the protein component of gintonin was identified in the Korea Basic Science Institute, Seoul (Ono et al. Mol. Cell Proteomics Sci. 5, 1338-1347, 2006; Gao et al., Mol. Cell Proteomics Sci. 7, 2399-2409, 2008).

The lyophilized peptides obtained above were reconstituted in 20 μL of 0.1% formic acid. Each sample was analyzed by carrying out independent experimental runs via LC/MS$^E$ using an ACQUITY ultra-pressure liquid chromatography (UPLC) and SynapQ-Time-of-Flight (TOF) mass spectrometer that was equipped with a Lockspray ion source (Waters, Manchester, UK).

Briefly, resuspended peptides (5 μL) in 0.1% formic acid was injected for 5 min at a flow rate of 10 μL/min to a Waters Symmetry C18 trapping column (180 μm i.d.x20 mm length with 5 μm particle size).

The peptides were separated in a pre-column by eluting with a liner gradient of 3-45% (w/v) B (A: 0.1%(w/v) formic acid in water, B: 0.1% (w/v) formic acid in acetonitrile) for 55 min under the condition of a 75 μm i.d.x250 mm length column packed with BEH130 C18 resin, and a 1.7 μm particle size (eluting rate; 280 nl/min). The column was rinsed for 25 min with 90% (w/v) B.

The column was re-equilibrated with 3% B for 20 min prior to the next run. All of the column temperatures were maintained at 35°C. The mass accuracy was maintained during the run by using a lockspray of the peptide [glu1] -fibrinopeptide B that was delivered through the auxiliary pump of a NanoACQUITY at 400 fmol/μm and 5 nl/min.

Peptides were analyzed in positive ion mode and the TOF analyzer was operated in V-mode with a typical resolving power of 10,000 fwhm. Prior to the analyses, the TOF analyzer was calibrated by using [glu1] -fibrinopeptide B fragments that were obtained using a collision energy of 30 eV and over the mass range 50-1990 m/z.

The Q-TOF was operated in the LC/ MS$^E$ acquisition mode. For each injection, the MS$^E$ mode was programmed to acquire data according to a suitable dual exact mass protocol.

ProteinLynx Global SERVER version 2.4 (PLGS 2.4) was used to process each of the raw data files obtained. Each processed data file were than searched against the *Apiales* protein database of UniProt (www.uniprot.org, May 3, 2011 Released version, number of entries 3,213)

Protein identification from the low/high collision spectra for each sample were processed using a hierarchical approach, which required detection of at least three fragment ion matches per peptide, seven fragment ion matches per protein, and two peptides per protein.

One of the most popular approaches of identifying proteins of plants including ginseng is a proteomic analysis using proteins extracted from electrophoresis bands (Nam et al., Proteomics 3, 2351-2367, 2003).

As can be seen in Table 1, IM-MS (Ion mobility-mass spectrometry) analysis identified two kinds of proteins from tryptic digests of the gintonin protein band obtained by electrophoresis (FIG. 1).

As for the protein component of gintonin, four peptide fragments are detected in the ginseng MLP151 while five are detected in the ribonuclease-like storage protein.

TABLE 1

| IM-MS Analysis Results and Amino Acid Sequences of Corresponding Proteins | | | |
|---|---|---|---|
| NW(KDa)/ PI | Amino Acid Sequence | Protein | Accession No. |
| 16.87/4. 9 | RDIEAHHLPK (SEQ ID NO: 1) | Ginseng major latex-like protein (MLP151) | EU939308 |
| | KDPTSYLDFLLSVTRD (SEQ ID NO: 2) | | |
| | KEEIVAIDEEDKS (SEQ ID NO: 3) | | |
| | KLNESVKD (SEQ ID NO: 4) | | |
| 27.3/5.5 | RSDYPWAM (SEQ ID NO: 6) | Ginseng ribonuclea se-like storage protein | AAR88098 |
| | KAFDIVGLLNQEGIYPNNDLYRPKM (SEQ ID NO: 7) | | |
| | KSLLNTFTIHGLYPYNAKG (SEQ ID NO: 8) | | |
| | KHLNAVPEIDFTKN (SEQ ID NO: 9) | | |
| | RTALAFRK (SEQ ID NO: 10) | | |

In other words, the protein component of gintonin is identified as ginseng MPL151 and ginseng RNAse-like storage protein, as measure by proteonomic analysis.

MLP151 and RNAse-like storage protein are known to have molecular weights of approximately 17 kDa and 27 kDa, respectively. In practice, however, the molecular weight of ginseng RNAse-like storage protein is often measured at 20~21 kDa as it is truncated at the C-terminus in ginseng (Kim et al., J Plant Physiol. 161, 837-845, 2004).

As shown in FIG. 1, gintonin is detected at approximately 13~15 kDa.

However, the protein component of gintonin is found to have a molecular weight of 17~27 kDa, as measured by proteomic analysis, which is discrepant to the electrophoretic measurement. This is attributed to the fact that gel migration is affected by sugars and charged components which are contained in gintonin, besides the protein component.

EXAMPLE 2: Assay for Expression of Membrane LPA Receptor Protein

The LPA receptor-null cell line B103 was cultured in DMEM supplemented with 10% heat-inactivated FBS and streptomycin/penicillin in a 5% $CO_2$/95% air incubator according to the method of Ishii et al. (Ishii et al., (2000) Mol Pharmacol 58. 895-902; Lee et al., (2006) J Biol Chem 281. 23589-23597; Yanagida et al., (2009) J Biol Chem 284. 17731-17741).

Human LPA (lysophosphatidic acid) receptor subtypes, orphan GPCRs (G protein coupled receptors) and plasmids carrying GPCRs were purchased from Missouri S&T resource Center (www.cDNA.org).

First, each of the plasmids carrying respective GPCRs was dissolved in a TE buffer, 1~100 ng of each plasmid was transfected into E. Coli (DH5$\alpha$) (ECOS, Intron) which was then smeared over LB plates containing ampicillin in petri dishes and cultured at 37°C overnight. Of the colonies thus grown in the petri dishes, 5~6 were inoculated into LB broth and cultured to amplify the plasmids. After being prepared using a DNA mini-preparation kit, the plasmids were digested and separated by electrophoresis to examine sizes of the cloned genes. When the correct genes were found to be cloned, the cell cultures were in part stored as stocks while the remainders were scaled up. By DNA maxi-preparation, cDNAs of LPA subtypes (LPA1 - LPA6) and other GPCR genes were obtained in sufficient amounts sufficient for transfection into B103 rat neuroblastoma cells.

The transient transfection of LPA receptor subtypes was carried out with the aid of Lipofectamine 2000 of Invitrogen. A plasmid (10.8 $\mu$g) carrying each LPA receptor (LPA1, LPA2, LPA3, LPA4, LPA5, and LPA6) was transfected into B103 cells grown in 10-cm dishes, and incubated for 2 days prior for use in experiments.

An experiment was carried out to examine whether the B103 cells transfected with the LPA receptors expressed LPA receptor proteins. In this regard, after an empty plasmid carrying none of LPA receptors, and plasmids carrying HA (hematoglutin)-LPA genes (Lee et al., J Biol Chem 281, 23589-23597, 2006) were transfected into LPA receptor-null B103 cells, cell membrane fractions from the cell homogenates were analyzed by Western blotting using an anti-HA antibody.

While the cells transfected with the empty plasmid devoid of LPA receptors did not express the LPA receptor proteins, the cells transfected with the plasmids carrying the LPA receptor genes expressed the LPA receptor proteins, indicating that gintonin acts on LPA receptor-expressing cells to induce an increase in intracellular calcium level (FIG. 2A).

In addition, the expression of LPA receptor proteins in LPA receptor-null B103 cells and B103 cells transfected with LPA receptor subtypes was examined by confocal laser microscopy.

For this, an empty plasmid containing none of the LPA receptors and plasmids carrying HA (hematoglutin)-LPA receptor genes (Ishii et al., Mol Pharmacol 58, 895-902, 2000; Lee et al., J Biol Chem 281, 23589-23597, 2006) were transfected into B103 cells which were then cultured to allow for the expression of the LPA receptors. Subsequently, an anti-HA primary antibody were reacted with cell membrane fractions and then labeled with a fluorescent Cy3-conjugated secondary antibody. Confocal laser microscopy (Fluoview FV1000, Olympus, USA) demonstrated the expression of LPA receptors in the cell membrane, as shown in FIG. 2B.

EXAMPLE 3: Effect of Gintonin on Intracellular Calcium and cAMP Level through Activation of LPA Receptors, Other Orphan GPCR Receptors and S1P1, S1P2 Receptors

Treatment with LPA induced a transient increase of intracellular free calcium ($[Ca^{2+}]_i$) in Fura 2AM-loaded B103 cells after an LPA receptor was transfected thereto, but did not induce prior to the transfection (Bandoh et al., J Biol Chem 274. 27776-27785, 1999; Im et al., Mol Pharmacol 57. 753-759, 1999).

In this Example, gintonin, or ginsenoside Rb1 or Rg1 was examined for its ability to induce a transient increase of $[Ca^{2+}]_i$ in Fura-2AM-loaded B103 cells which were tranfected with LPA receptors and in Fura-2AM-loaded B103 cells which were not transfected with LPA receptors. To this end, B103 cells ($2~4 \times 10^6$/ml) pre-treated with Fura-2AM were suspended in a 1.5 mM $Ca^{2+}$-containing buffer or $Ca^{2+}$-free buffer, and incubated 37°C for 30 min, followed by analyzing intracellular calcium levels in the B103 cells in the presence of gintonin or ginsenosides.

However, the effect of gintonin on the increase of $[Ca^{2+}]_i$ in B103 cells (ATTC Cell Bank, USA) was examined with crude gintonin due to a limited amount of available individual pure gintonins.

Briefly, B103 cells ($1~2 \times 10^6$/ml) were mixed with 2.5 $\mu$M Fura 2-AM in a $Ca^{2+}$ buffer (pH 7.4) containing NaCl 120 mM, KCl 5 mM, $MgCl_2$ 1 mM, $CaCl_2$ 1.5 mM, glucose 10 mM, and HEPES 25 mM, and a $Ca^{2+}$ free buffer (pH 7.4) containing NaCl 120 mM, KCl 5 mM, $MgCl_2$ 1 mM, EGTA 0.2 mM, glucose 10 mM, and HEPES 25 mM at 37°C for 30 min in water bath with shaking according to the Jørgensen method(Jørgensen N.K., Petrson S.F. and Hoffman E.K. Am J Physiol Cell Physiol, 276, 26-37, 1999), and washed three times with $Ca^{2+}$ buffer or $Ca^{2+}$ free buffer to remove excess Fura-2AM.

$[Ca^{2+}]_i$ was estimated in Fura 2AM-loaded cells in suspension using an RF-5300PC intracellular ion measurement system (Shimadzu Corporation, Japan).

Briefly, Fura 2-AM loaded cells were diluted with an experimental medium to a final density of $2{\sim}4 \times 10^6$ cells/mL, and transferred to polystyrene cuvettes (Elkay Ultra-VU). The cells were stirred using Teflon-coated magnets, and the cuvette housing was thermostatically controlled at 37°C. The excitation wavelengths were alternated between 340 and 380 nm under computer control. Emission was detected at 510 nm. Excitation and emission slit widths were 5 nm. Background calibration was performed as described by Jørgensen et al.

Measurements of 340 nm vs. 380 nm ratio values were converted into $[Ca^{2+}]_i$ values using the formula of Hounsell et al. (Hounsell, E.F., Davies, M.J., and Smith, K.D., Protein protocol handbood, Humanna press, Totawa, 803-804, 1997).

$$[Ca^{2+}] = K_d \left[ (R - R_{min}) / (R_{max} - R) \right] (S_{f380}/S_{b380})$$

wherein *Kd* is the effective dissociation constant (224 nM), R is a fluorescence ratio of measurements of fluorescence at 340 nm to at 380 nm, and $R_{max}$ and $R_{min}$ are R values measured at a saturated concentration with 50 $\mu$g/ml digitonin and in a free medium with 20 mM EGTA, respectively. $S_{f380}$ and $S_{b380}$ represent fluorescence intensities at 380 nm in the presence of digitonin and EGTA, respectively, and when these values are represented, the ratio thereof is of maximum and minimum values (Grynkiewicz, G., M. Poenie, and R. Y. Tsien, J Biol Chem260: 3440-3450, 1985).

As described above, an examination was made of the effect of gintonin on intracellular calcium ($[Ca^{2+}]_i$) and cAMP changes in null B103 cells before and after LPA receptor subtypes were transfected thereto. As can be seen in FIG. 3A, gintonin almost did not induce an increase in intracellular calcium level in LPA receptor-null B103 cells transfected with a plasmid carrying no LPA receptor genes (empty vector) (intracellular calcium level increased by 22.7±4.1 nM). Meanwhile, gintonin was applied to B103 cells which were transfected with and thus expressed LPA receptor subtypes (LPA1-LPA6). Gintonin (1 $\mu$g/ml) were observed to increase an intracellular calcium level by 185.9±12.8 in LPA1-expressing B103 cells, by 208.3+13.0 in LPA2-expressing B103 cells, by 206.5+11.2 in LPA3-expressing B103 cells, and by 297.5+21.8 nM in LPA5-expressing B103 cells. Upon treatment with gintonin, LPA4-expressing B103 cells increased in intracellular calcium level only by 61.6±11.8 nM, indicating that gintonin acts on LPA4 receptor to a lesser extent than the other LPA receptors. No gintonin activity was detected with regard to LPA6 receptor.

The activation of LPA receptors is associated not only with an increase in intracellular calcium level, but also with the inhibition or stimulation of intracellular cAMP formation (Ishii et al., Mol Pharmacol 58, 895-902, 2000; Contos et al., Mol Pharmcol 58, 1188-1196, 2000; Kimura et al., J Biol Chem. 276, 15208-15215. 2001). Particularly, activated LPA6 receptor is reported to be implicated in the formation of intracellular cAMP, but not associated with the increase of intracellular cAMP (Yanagida et al., J Biol Chem 284, 17731-17741, 2009).

After treatment with gintonin, B103 cells expressing LPA3 or LPA6 receptor were examined for change in intracellular cAMP level. Gintonin was observed to have no influences on intracellular cAMP level (FIG. 4C).

Therefore, gintonin-induced LPA receptor activation is associated predominantly with the increase of intracellular calcium level.

In addition, GPR35 (Oka et al., Biochem Biophys Res Commun. 395, 232-237, 2010) and GPR87 (Tabata et al., Biochem Biophys Res Commun. 363, 861-866, 2007), both known as orphan GPCRs reacting with LPA, and GPR40, GPR41, GPR43 and GPR120 (Hirasawa et al., Biol Pharm Bull. 31, 1847-1851, 2008), all known as fatty acid receptors reacting with free fatty acids, were expressed in B103 cells where gintonin activity was tested.

As can be seen in FIG. 3B, gintonin was observed to increase an intracellular calcium level in GPR35-expressing B103 cells, but slightly, and to not act on the other receptors. These results suggest the selectivity of gintonin for LPA receptor subtypes with regard to the increase of intracellular calcium levels.

EXAMPLE 4. Change of Intracellular Free $Ca^{2+}$ Level in LPA Receptor-Expressed Cell with LPA and Gintonin

To obtain the concentration-response curve in the presence of gintonin, the observed peak amplitudes were normalized and plotted, and then fitted to the following Hill equation below using Origin software (Northampton, MA):

$$y/y_{max} = [A]^n / ([A]^n + [EC_{50}]^n),$$

wherein y is the percentage activity at a given concentration of gintonin, $y_{max}$ is the maximal peak current, $[EC_{50}]$ is the concentration of gintonin producing half-maximum effect of the control response to the gintonin, $[A]$ is the concentration of gintonin, and $n$ is the interaction coefficient. All values are presented as means $\pm$ S.E. The differences between means of the control and gintonin treatment data were analyzed using an unpaired Student's $t$-test. A value of $p < 0.05$ was considered statistically significant.

LPA receptor-null B103 rat neuroblastoma cells (Valentine et al., Biochim Biophys Acta 1780, 597-605, 2008) were transfected with a plasmid carrying no LPA receptor genes, or a plasmid carrying an LPA3 receptor gene, and cultured to express LPA3 receptor or not. To verify whether LPA3 receptor was normally expressed in the cells, and whether the LPA3 receptors expressed in the cells responded to LPA, the transfected B103 cells were examined for intracellular calcium level after treatment with the ligand of LPA receptors. As a result, LPA increased intracellular calcium levels in the LPA3 receptor-expressing B103 cells in a dose-dependent manner. LPA had an $EC_{50}$ of $58.9\pm5.54$ nM for the cells (FIG. 4A).

On the other hand, LPA did not induce the increase of intracellular calcium level in B103 cells which did not express LPA3 receptor (FIG. 4A).

Also, the gintonin-induced increase of intracellular calcium level in LPA receptor subtypes (LPA1-LPA6) -expressing B103 cells was investigated. With regard to LPA receptor-mediated intracellular calcium increased, as can be seen in FIG. 3B, gintonin had an $EC_{50}$ of $0.10\pm0.02$ for LPA1 receptor, $0.0040\pm0.0004$ for LPA2 receptor, $0.12\pm0.01$ for LPA3 receptor, and $0.046\pm0.003$ nM for LPA5 receptor.

Particularly, the much lower $EC_{50}$ for LPA2 indicates the higher affinity of gintonin for LPA2 receptor than the other LPA receptor subtypes.

EXAMPLE 5. Comparison of Intracellular Free $Ca^{2+}$ Increase through LPA Receptor Activationbetween Gintonin and Ginsenosides Rb1, Rg1 and Rg3

To examine whether ginsenosides, which are known as physiologically/pharmaceutically effective ingredients of ginseng (Nah et al., CNS Drug Rev. 13, 381-404, 2007), can induce an increase in intracellular calcium level through LPA receptor activation, most abundant and representative ginsenosides RB1, Rg1 and Rg3 were taken for comparison with gintonin.

Treatment with LPA induced a transient increase of intracellular free calcium ($[Ca^{2+}]_i$) in Fura 2AM-loaded B103 cell s after an LPA receptor was transfected thereto, but did not induce prior to the transfection (Bandoh et al., J Biol Chem 274. 27776-27785, 1999; Im et al., Mol Pharmacol 57. 753-759, 1999).

In this Example, gintonin, or ginsenoside Rb1 or Rg1 was examined for its ability to induce a transient increase of $[Ca^{2+}]_i$ in Fura-2AM-loaded B103 cells which were tranfected with LPA receptors and in Fura-2AM-loaded B103 cells which were not transfected with LPA receptors. To this end, B103 cells ($2\sim4\times10^6$/ml) pre-treated with Fura-2AM were suspended in a 1.5 mM $Ca^{2+}$-containing buffer or $Ca^{2+}$-free buffer, and incubated 37°C for 30 min, followed by analyzing intracellular calcium levels in the B103 cells in the presence of gintonin or ginsenosides.

However, the effect of gintonin on the increase of $[Ca^{2+}]_i$ in B103 cells (ATTC Cell Bank, USA) was examined with crude gintonin due to a limited amount of available individual pure gintonins.

Briefly, B103 cells ($1\sim2\times10^6$/ml) were mixed with 2.5 $\mu$M Fura 2-AM in a $Ca^{2+}$ buffer (pH 7.4) containing NaCl 120 mM, KCl 5 mM, $MgCl_2$ 1 mM, $CaCl_2$ 1.5 mM, glucose 10 mM, and HEPES 25 mM, and a $Ca^{2+}$ free buffer (pH 7.4) containing NaCl 120 mM, KCl 5 mM, $MgCl_2$ 1 mM, EGTA 0.2 mM, glucose 10 mM, and HEPES 25 mM at 37°C for 30 min in water bath with shaking according to the Jørgensen method (Jørgensen N.K., Petrson S.F. and Hoffman E.K. Am J Physiol Cell Physiol, 276, 26-37, 1999), and washed three times with $Ca^{2+}$ buffer or $Ca^{2+}$ free buffer to remove excess Fura-2AM.

$[Ca^{2+}]_i$ was estimated in Fura 2AM-loaded cells in suspension using an RF-5300PC intracellular ion measurement system (Shimadzu Corporation, Japan).

Briefly, Fura 2-AM loaded cells were diluted with an experimental medium to a final density of $2\sim4x10^6$ cells/mL, and transferred to polystyrene cuvettes (Elkay Ultra-VU). The cells were stirred using Teflon-coated magnets, and the cuvette housing was thermostatically controlled at 37°C. The excitation wavelengths were alternated between 340 and 380 nm under computer control. Emission was detected at 510 nm. Excitation and emission slit widths were 3 nm. Background calibration was performed as described by Jørgensen $et$ $al$. Digitonin and EGTA were used as concentration adjustment reagents to make a condition in which fura-2AM completely combines with and disassociates from $Ca^{2+}$.

Measurements of 340 nm vs. 380 nm ratio values were converted into $[Ca^{2+}]_i$ values using the formula of Hounsell et al. (Hounsell, E.F., Davies, M.J., and Smith, K.D., Protein protocol handbood, Humanna press, Totawa, 803-804, 1997).

$$[Ca^{2+}] = K_d \ [(R - R_{min})/(R_{max} - R)] \ (S_{f380}/S_{b380})$$

wherein Kd is the effective dissociation constant (224 nM), R is a fluorescence ratio of measurements of fluorescence at 340 nm to at 380 nm, and $R_{max}$ and $R_{min}$ are R values measured at a saturated concentration with 50 $\mu$g/ml digitonin and in a free medium with 20 mM EGTA, respectively. $S_{f380}$ and $S_{b380}$ represent fluorescence intensities at 380 nm in the presence of digitonin and EGTA, respectively, and when these values are represented, the ratio thereof is of maximum and minimum values (Grynkiewicz, G., M. Poenie, and R. Y. Tsien. J Biol Chem260: 3440-3450, 1985).

To obtain the concentration-response curve in the presence of gintonin, the observed peak amplitudes were normalized and plotted, and then fitted to the following Hill equation below using Origin software (Northampton, MA):

$$y/y_{max} = [A]^n/([A]^n + [EC_{50}]^n),$$

wherein $y$ is the percentage activity at a given concentration of gintonin, $y_{max}$ is the maximal peak current, $[EC_{50}]$ is the concentration of gintonin producing half-maximum effect of the control response to the gintonin, $[A]$ is the concentration of gintonin, and $n$ is the interaction coefficient. All values are presented as means $\pm$ S.E. The differences between means of the control and gintonin treatment data were analyzed using an unpaired Student's $t$-test. A value of $p < 0.05$ was considered statistically significant.

As can be seen in FIG. 5A, gintonin increased intracellular calcium levels by activating LPA3 receptor whereas no increased intracellular calcium levels were detected in the cells treated with Rb1, Rg1, and Rg3 (each 10 $\mu$M), demonstrating that the ginsenosides do not induce the activation of the LPA receptors (FIG. 4B). In addition, gintonin was observed to induce an increase in intracellular calcium level by activating LPA receptors in spite of co-existence with the ginsenosides.

EXAMPLE 6: Effect of the LPA Receptor Antagonist Kil6425 on Gintonin-Induced LPA Receptor Activation

Given the assumption that gintonin might bind to and thus activate LPA receptors to induce an increase in intracellular calcium level, an LPA receptor antagonist is anticipated to block the function of gintonin. To confirm this, the LPA receptor antagonist Kil6425 was employed together with gintonin.

As is apparent from the data of FIG. 7, when cells expressing LPA1 and LPA3 receptors were treated with Ki16425, which antagonizes LPA1 and LPA 3 receptors (Ohta et al., Mol Pharmacol 64. 994-1005, 2003), the cells did not increase in intracellular calcium level even in the presence of gintonin, demonstrating that the gintonin-induced intracellular calcium increase is mediated by LPA receptors.

EXAMPLE 7: Effect of Extracellular Calcium and $Ca^{2+}$ Chelator BAPTA on Gintonin-Induced Activation of LPA Receptor

When bound by a ligand, most $G\alpha_{q/11}$-binding protein-coupled receptors (GPCRs) transmit the signaling of increasing intracellular calcium levels. In this regard, the calcium may be sourced from the extracellular space or from the ER. That is, when activated, the receptors open calcium channels to induce calcium influx from the extracellular space, or the binding of $IP_3$ to its receptor $IP_3R$ stimulates release of calcium from ER, a calcium reservoir (Berridge et al., Nature 395. 645-648, 1998).

In this Example, an examination was made of the source from which the calcium contributing to the gintonin-induced increase of intracellular $Ca^{2+}$ level comes from.

As a result, the transient increase of $[Ca^{2+}]_i$ by gintonin-induced LPA receptor activation in cells expressing LPA1 and LPA3 receptors was observed, but at a greatly lowered level, when extracellular $Ca^{2+}$ was depleted (a $Ca^{2+}$-free buffer containing 0.2 mM EGTA) (FIG. 7), indicating that extracellular $Ca^{2+}$ is a source of the intracellular calcium influx upon gintonin-induced LPA receptor activation.

On the other hand, the observation of the transient increase of $[Ca^{2+}]_i$, although at a very low level, in the absence of extracellular $Ca^{2+}$ suggests the existence of an intracellular $Ca^{2+}$ source. In addition, the gintonin-induced increase of intracellular calcium level in LPA receptor-expressing cells became invalid in the cells pretreated with the intracellular $Ca^{2+}$ chelator BAPTA (FIG. 7).

Taken together, the data obtained above demonstrate that gintonin activates LPA receptors, which, in turn, induces an intracellular calcium increase, whether from the extracellular space or an intracellular source.

EXAMPLE 8: Signaling of Gintonin-Induced LPA Receptor Activation

LPA-induced LPA receptor activation is transmitted differently depending on LPA receptor-expressing cells, for example, via either, both, or a combination of pertussis toxin (PTX)-sensitive GTP-binding protein ($G\alpha_{i/o}$), and PTX-

insensitive GTP-bindingn ($G\alpha_{q/11}$ or $G\alpha_{12/13}$) (An et al., Mol Pharmacol 54, 881-886, 1998; Yoshida and Ueda, Jpn J Pharmacol 87, 104-109, 2001).

Based on the observation, an experiment was conducted to see whether the increase of intracellular calcium level resulting from the activation of LPA receptor by gintonin is mediated by pertussis toxin (PTX)-sensitive GTP-binding protein. As can be seen in FIG. 6, the gintonin-induced increase of intracellular calcium level was significantly lowered by 25 % in LPA3 receptor-expressing B103 cells pre-treated with PTX (200 ng/ml, 16 hrs), compared to cells not treated with PTX.

This result suggests that the increase of intracellular calcium level resulting from the activation of LPA receptor by gintonin is mediated predominantly through PTX-insensitive $G\alpha_{q/11}$ or $G\alpha_{12/13}$ protein, but $G\alpha_{i/o}$ protein is also, in part, involved in the gintonin function (FIG. 6 B).

As a rule, when stimulated, $G\alpha_{q/11}$-binding protein-coupled receptors (GPCRs) activate phospholipase C (PLC), which, in turn, generates diacylglycerol (DAG) and $IP_3$. DAG is required to activate protein kinase C (PKC), and $IP_3$ binds to endoplasmic reticulum (ER)-resident $IP_3$ receptor to induce the release of calcium from ER, a reservoir of calcium, into the cytoplasm (Berridge et al., Nature 395. 645-648, 1998).

In this Example, an examination was made in LPA2-expressing B103 cells to see whether the gintonin-induced increase of intracellular calcium level takes the signal transduction pathway of PLC.

As can be seen in FIG. 7, the action of gintonin was blocked not only by the active PLC inhibitor U73122, but also by the $IP_3$ receptor antagonist 2-APB, demonstrating that gintonin-triggered intracellular calcium increase takes the following signaling pathway: binding to LPA receptor - $G\alpha_{q/11}$-binding protein activation -PLC activation - DAG and $IP_3$ generation - $IP_3$ receptor activation - intracellular calcium increase.

In addition, when LPA receptor-expressing B103 cells are pre-treated with the PKC activator PMA to activate PKC in advance (Urs et al., J Biol Chem. 283, 5249-5257. 2008), gintonin-induced intracellular calcium increase was blocked, implying that PKC is involved in the action of gintonin (data not shown).

EXAMPLE 9: Influence of Mutant LPA Receptor on Gintonin Function

When LPA is applied to B103 cells transfected with mutant LPA receptors which have substitution mutations at specific residues of the LPA binding site, the action of LPA is significantly reduced or vanished (Valentine et al., J. Biol. Chem 283, 12175-12187, 2008).

That is, LPA receptors have specific amino acid residues, known as binding sites, which are responsible for interaction with LPA, and the binding of LPA to the specific amino acids starts to exert the function. This Example was configured to examine a change in the gintonin-induced intracellular calcium increase through LPA receptor activation in LPA3 receptor-expressing cells when specific amino acids of the LPA binding site in LPA receptors were mutated.

To this end, a mutant LPA receptor in which Arg3.28, known as a common LPA binding site of LPA1-LPA3 receptors, was mutated as Arg3.28Ala (that is, Arg is substituted by Ala; R3.28A) or Trp4.64, known as an LPA-binding site of LPA3 receptor was mutated as Trp4.64A1a (that is, Trp is substituted by Ala; W4.64A) (Valentine et al., J. Biol. Chem. 283, 12175-12187, 2008) was transfected to B103 cells which were then tested for the action of gintonin, in comparison with cells transfected with the wild-type. As can be seen in FIG. 8, gintonin-induced intracellular calcium increase was observed in the wild-type whereas the gintonin action was greatly reduced in the mutant LPA receptor-transfected cells. From the result, it is henceforth understood that the gintonin-triggered LPA receptor activation is achieved by interaction with the LPA binding sites of LPA receptors and that gintonin occupies the LPA binding sites to activate LPA receptors.

EXAMPLE 10: Activation of NMDA Receptor by Gintonin

The NMDA (*N*-methyl *D*-aspartate) receptor is an ionotropic glutamate receptor, abundantly found in the central nervous system, inter alia, in the hippocampus, containing both a ligand binding site and an ion channel. Activation of NMDA receptors results in the opening of an ion channel that is non-selective to cations. This receptor is characterized by voltage-dependent activation, a glycine-binding site, and a result of ion channel block by extracellular $Mg^{2+}$. Hence, when activated, the NMDA (*N*-methyl *D*-aspartate) receptor allows the flow of $Na^+$ and $Ca^{2+}$ into cells and $K^+$ out of the cells, giving rise to post-synaptic depolarization on neurons (Dingledine et al., Pharmacol. Rev. 51, 7-61. 1999; Cull-Candy et al., Curr. Opin. Neurobiol. 11, 327-335, 2001; Paoletti and Neyton, Curr Opin Pharmacol 7, 39-47, 2007).

In addition, the NMDA receptor, distributed in the central nervous system, inter alia, the hippocampus, plays a critical role in learning and memory because the $Ca^{2+}$ influx through NMDA receptors induces long-term potentiation (LTP) closely associated with synaptic plasticity, a cellular mechanism for learning and memory (Purves, Dale, George J. Augustine, David Fitzpatrick, William C. Hall, Anthony-Samuel LaMantia, James O. McNamara, Leonard E. White Neuroscience, 4th Ed.. Sinauer Associates. pp. 191-195. http://www.sinauer.com/neuroscience4e. synaptic plasticity).

This Example is configured to examine whether gintonin activates NMDA receptors. Activity of NMDA receptors was measured in *Xenopus* oocytes expressing NMDA receptors, as follows.

10-(1). Preparation of NMDA Receptor Gene and cDNA

cDNAs of NMDA receptor subunits (NR1 and NR2) were employed (Zheng et al., J Neurosci. 17, 8676-8686, 1997). cDNAs (100 ng/40 nl) corresponding to respective NMDA receptor subunits (NR1 and NR2) were injected into animal or vegetal poles of oocytes by use of a 10 ml-microdispenser (VWR Scientific, USA) and incubated for 4-5 days after injection (Zheng et al., J Neurosci. 17, 8676-8686, 1997).

10-(2). Oocyte Preparation

*Xenopus laevis* frogs were obtained from Xenopus I (Ann Arbor, MI). Their care and handling were in accordance with the highest standards of institutional guidelines. To isolate oocytes, the frogs were operated on under anesthesia with an aerated solution of 3-aminobenzoic acid ethyl ester. Oocytes were separated by treatment with collagenase and agitation for two hours in a $Ca^{2+}$-free medium containing 82.5mM NaCl, 2 mM KCl, 1 mM $MgCl_2$, 5 mM HEPES, 2.5 mM sodium pyruvate, 100 units/ml penicillin and 100 $\mu$g/ml streptomycin.

Stage V-VI oocytes were collected and stored in ND96 (96 mM NaCl, 2 mM KCl, 1 mM $MgCl_2$, 1.8 mM $CaCl_2$, and 5 mM HEPES, pH 7.4) supplemented with 50 $\mu$g/ml gentamicin. This oocyte containing solution was maintained at 18°C with continuous gentle shaking while the ND96 medium was changed to a fresh one every day.

10-(3). Measurement of NMDA Receptor Activity

Two-electrode voltage-clamp recordings were obtained from individual oocytes placed in a small Plexiglas net chamber (5 ml). Electrophysiological experiments were performed using microelectrodes filled with 3 M KCl (resistance of 0.2-0.7 M$\Omega$) and an Oocyte Clamp amplifier (OC-725C; Warner Instrument, Hamden, CT). For physiological recordings of NMDA receptor activity, oocytes were first perfused with $Mg^{2+}$-free ND96 (mM: 96 NaOH, 2 KOH, 0.3 $CaCl_2$, 5 HEPES pH 7.6 with methanesulfonic acid). Then, the oocytes were clamped at -60 mV holding potential, followed by recoding NMDA currents (induced by administering 300 mM NMDA + 10 mM glycine) (Zheng et al., J Neurosci. 17, 8676-8686, 1997; Chang and Kuo, J Neurosci. 28, 1546-1556, 2008).

As can be seen in FIG. 9A, treatment with NMDA induced an inward current in NMDA receptor-expressing oocytes, indicating the successful expression of NMDA receptors. From the data of FIGS. 9B and 9C, it was observed that gintonin induced NMDA receptor-mediated inward currents in a concentration-dependent manner in Gintonin NMDA receptor-expressing *Xenopus* oocytes, with an $ED_{50}$ detected at 0.49$\pm$0.10 $\mu$g/ml.

Further, there was the likelihood that the NMDA receptor activation by gintonin in Xenopus oocytes might be transmitted via the G protein-PLC-$IP_3$-$Ca^{2+}$ pathways when gintonin binds to LPA1 receptor endogenous to Xenopus oocytes (Kimura et al., J Biol Chem. 276, 15208-15215, 2001). For this, an experiment was carried out to see whether gintonin-induced NMDA receptor activation is blocked by an LPA receptor antagonist.

As can be seen in FIG. 10A, treatment with Ki16425 (10 $\mu$M), known as an antagonist to LPA1 and LPA3 receptors (Ohta et al., Mol Pharmacol 64. 994-1005, 2003), had no influences on NMDA-NMDA receptor activation, but reduced gintonin-induced CaCC activation, and blocked gintonin-induced NMDA receptor activation with a statistical significance (*p<0.001, compared to control), confirming that gintonin activates endogenous LPA receptors to elicit NMDA receptor activation (FIG. 10B).

Moreover, an examination was made of the pathway through which gintonin signaling is mediated to NMDA receptor activation. Treatment with the active PLC (phospholipase C) inhibitor U73122 (1 $\mu$M) did not block NMDA-induced inward current, but suppressed gintonin-induced CaCC activation and blocked gintonin-induced NMDA receptor activation (FIG. 11A). In contrast, the inactive phospholipase C (PLC) inhibitor U73343 (1 $\mu$M) did not block gintonin-induced NMDA receptor activation (data not shown).

Likewise, the $IP_3$ receptor antagonist 2-APB did not block NMDA-induced inward current, but suppressed gintonin-induced CaCC activation and blocked gintonin-induced NMDA receptor activation (FIG. 11 B).

In addition, when treated with the intracellular calcium chelator BAPTA-AM, the Xenopus oocytes showed normal NMDA-induced inward currents, but a reduction in gintonin-induced CaCC activation, and NMDA receptor activation (FIG. 11C). These data confirmed that gintonin induces the activation of NMDA receptors via the LPA1 receptor endogenous to Xenopus oocytes (Kimura et al., J Biol Chem. 276, 15208-15215, 2001) and its downstream pathways PLC-$IP_3$-$Ca^{2+}$, but not via the NMDA pathway (FIG. 11D).

An increase in intracellular calcium level incites protein kinase C (PKC) to phosphorylate NMDA receptors. That is, the NMDA receptor has a phosphorylation site, and is activated by PKC (Urushihara et al., J Biol Chem 267, 11697-11700, 1992; Zheng et al., J Neuroscience 15, 8676-8686, 1997; Lia et al., Mol Pharmaocol 59, 960-964, 2001). In addition, the signaling of the $G\alpha_{q/11}$ protein-coupled receptor is mediated to the NMDA receptor via the signaling pathway of PKC, tyrosine kinase and Src-family tyrosine kinase (Lu et al., Nature neuroscience 2, 331-338, 1999).

In the present invention, an examination was made of kinases and protein phosphatases which are involved in the downstream pathway through which gintonin-induced NMDA receptor activation is mediated.

PKC, tyrosine kinase and Src-family kinase were identified as kinase and tyrosine phosphatase in the present invention. Also, receptor protein tyrosine phosphatase (RPTP) $\alpha$ was identified as a protein phosphatase. Upon PLC activation, diacyl glycerol (DAG) is produced, together with $IP_3$. DAG is known as an activator of PKC. RPTP is an enzyme which plays an important role in the regulation of $G\alpha_{q/11}$ protein-coupled receptor-mediated Src-family kinase and tyrosine kinase activities (Tsai et al., EMBO J, 18, 109-118, 1999; Petrone et al., EMBO J 22, 4121-4131, 2003).

Next, the roles of tyrosine kinase, Src-family kinase, and receptor protein tyrosine (RPTP) in the NMDA receptor activation caused by gintonin were studied.

As can be seen FIG. 12A, cells expressing a mutant NMDA receptor (S1308A) in which the serine at position 1308 (S1308), known as a PKC phosphorylation site, was substituted by alanine, exhibited a low level of gintonin-induced NMDA receptor activation whereas cells expressing a mutant NMDA receptor (S1312A) maintained gintonin-induced NMDA receptor activation. Thus, it is understood that gintonin stimulates PKC to phosphorylate the NMDA receptor at a specific site (S1308), and thus that gintonin-induced NMDA receptor activation is associated with PKC activity.

Genistein (10 $\mu$M), known as a tyrosine kinase inhibitor, was observed to inhibit gintonin-induced NMDA receptor activation, showing that gintonin causes the activation of tyrosine kinase, which, in turn, activates the NMDA receptor by phosphorylating a specific tyrosine residue (FIG. 12B).

In addition, PP2 (30 $\mu$M), known as an active inhibitor of Src-family kinase, blocked the action of gintonin while PP3 (30 $\mu$M), known as an inactive inhibitor, did not show inhibitory activity. Thus, the gintonin-induced NMDA receptor activation is proven as requiring the activation of Src-family kinase as a mediator (FIG. 12C).

Moreover, oocytes expressing active or inactive phosphoprotein phosphatase (RPTP) $\alpha$ exhibited NMDA receptor activity at a low level when treated with gintonin (FIG. 12D).

Therefore, gintonin stimulates various kinases to activate NMDA receptors by phosphorylation, but RPTP blocks the phosphorylation of NMDA receptors, thus inhibiting gintonin-induced NMDA receptor activation. So, when Xenopus oocytes are treated with gintonin, LPA receptors endogenous to Xenopus oocytes are activated to cause an elevated intracellular calcium level through the $PLC-IP_3-Ca^{2+}$ pathway, which, in turn, induces the calcium-dependent activation of PKC, tyrosine kinase and Src-family protein, resulting in the activation of NMDA receptors.

EXAMPLE 11. Effect of Gintonin on Induction of Long-Term Potentiation (LTP) in Rat Hippocampal Slice

Long-term potentiation (LTP) is a long-lasting enhancement in signal transmission between two neurons that results from stimulating them synchronously (Cooke and Bliss, Brain 129, 1659-1673, 2006). It is one of several phenomena underlying synaptic plasticity. As memories are thought to be encoded by modification of synaptic strength, LTP is widely considered to be one of the major cellular mechanisms underlying learning and memory (Bliss and Collingridge, Nature 361, 31-39, 1003).

LTP is discovered mostly in the hippocampus responsible for learning and memory. For studying synaptic transmission, LTP can be induced by tetanic stimulation. This is a model studied by an activity-dependent change in synaptic strength accounting for storing information in the brain. During tetanic stimulation, large and long depolarization occurs, giving rise to inducing NMDA receptor activation, with the consequent production of a serial of processes of increasing permeability to $Ca^{2+}$, allowing for $Ca^{2+}$ influx into cells through NMDA receptor channels, and enhancing synaptic efficacy. For this reason, $Ca^{2+}$ ions influent into cells through activated NMDA receptor channels switch its role to the onset of LTP.

NMDA-receptor-dependent synaptic plasticity is regarded as a cellular basis for learning and memory.

Since gintonin induces NMDA receptor activation as confirmed above, an examination was made of the effect of gintonin on LTP induction in rat hippocampal slices (Moon et al., Neursci. Lett. 466, 114-119, 2009; Lee et al., J Neurosci. Res. 89, 96-107, 2011). In this regard, LTP induction was studied after treatment with three different concentrations of gintonin (0.1, 1, and 10 $\mu$g/ml).

11-(1). Preparation of rat hippocampal slices

Male rats 3~5 weeks old (Sprague-Dawley strain)(Charls River, U.S.A.) were quickly sacrificed by cervical dislocation without using an anesthetic (Steidl et al., Brain Res. 1096, 70-84, 2006). With the aid of a rongeur (Fine Science Tools Inc., USA), the cranium was removed, and the brain was excised, immediately cooled in ice, and immersed in artificial cerebrospinal fluid (saCSF) containing oxygen-saturated (95% $O_2$/5% $CO_2$) sucrose (composition mM: Sucrose 248, $NaHCO_3$ 26, glucose 10, KCl 3, $CaCl_2$ 2, $MgCl_2$ 1, HEPES 10, pH7.4).

The hippocampus was sectioned into 400 $\mu$m slices using vibratome (MA752 motorised advance vibroslice; Campden inc.) and the hippocampal slices were stabilized in artificial cerebrospinal fluid (aCSF) (composition mM:

NaOH 124, NaHCO$_3$ 26, Glucose 10, KCl 3, CaCl$_2$ 2, MgCl$_2$ 1, HEPES 10, pH 7.4) for 1 hr (Lelong and Rebel, J Pharmacol Toxicol Methods. 39, 203-210, 1998).

11-(2). Organotypic hippocampal slice (OHSC) incubation

OHSCs were incubated accordint to the Stoppini method (Stoppini et al., J Neurosci Methods 37, 173-182, 1991), and all procedures were conducted on a sterilized bench.

Immediately after excision from the cranium, the rat brain was immersed in an ice-cold HBSS-medium (LB 003-01, Sigma, St.Louis, Mo, USA), and the hippocampus was separated and sectioned at a thickness of 400 μm using a tissue chopper (Mickle Laboratory Engineering Co., Surrey, UK). The hippocampal slices were placed on a membrane insert (polytetrafluorethlene membranes, 0.4 μm, Millicell-CM, Millipore Co., Bedford, MA, USA), and immersed in a culture medium.

The medium was based on a 50% MEM-medium (LM 007-01, JBI, Daegu, South Korea), 25% horse serum (S 104-01, Daegu, South Korea), a 25% Hank's balanced salt solution (LB 003-1, JBI, Daegu, South Korea), 6 g/ℓ D-glucose (G-7528, Sigma, St. Louis, MO, USA), 1 mM L-glutamine (G-8540, Sigma, St. Louis, MO, USA), 20 mM HEPES(H-4034, Sigma, St. Louis, MO, USA) and 1% penicillin-streptomycin (LS 202-02, Gibco BRL, USA), pH 7.1. Before use in experiments, the hippocampal slices were cultured for 14 days in an incubator (36°C, 95% O$_2$, 5% CO$_2$), with the culture medium exchanged with a fresh one every 2~3 days.

11-(3). Electrophysiological recording

1) Preparation of hippocampal slices on MEA probes

Before use, MEA probes (Multi channel system GmbH, Germany; each electrode: 30×30 μm, distance: 200 μm) were coated with 0.1% polyetherimide (PEI, Sigma, St.Louis, MO, USA), and dried under UV light for 90 min on a sterilized bench. The probes were washed once or twice with secondary distilled water. The hippocampal slices were removed from the membrane inserts and then placed on probes of a multielectrode array. Each slice was positioned, and the surrounding solution was removed using a pipette. A fresh aCSF solution was allowed to flow over the slices such that they were stabilized in the solution.

The MEA containing the hippocampal slice was transferred to an MEA1060 amplifier interface (Multi Channel Systems GmbH, Germany), and after the slices were warmed to 32°C under the control of the temperature controller (Multi Channel System GmbH, Germany), stimuli were allowed to reach the slices. The solution in the array was grounded using an Ag/AgCl pellet.

2) Induction of long-term potentiation (LTP)

Bipolar constant current pulses were produced using the data acquisition software through a digital stimulator with a built-in isolator (Multi Channel Systems GmbH, Germany). To collect typical responses, one of the electrodes in the Schaffer collateral fibers area was selected as a stimulating electrode position while another one in the stratum radiatum of the Cornu ammonis 1 was selected as a recording electrode position (Shimono et al., Neural Plast. 9(4), 249-254, 2002).

LTP was induced using standard protocols, which had a 100-Hz theta burst stimulation or a tetanic train stimulation containing 2 bursts of 1 sec at 100 Hz with 30-sec intervals between each burst. Field potential (FP) recordings after LTP induction were performed for an additional 120 min period every 30 sec to record the LTP condition. Stimulation and recording were carried out using the Recorder-Rack software (Multi Channel Systems GmbH, Germany).

3) Treatment with gintonin

During the LTP experiment, the sliced were continuously perfused at a rate of 1 ml/min with an aCSF solution containing 95% O$_2$ and 5% CO$_2$. The time schedules for a normal condition free of theta burst stimulation (TBS), for TBS alone, and for TBS in combination with gintonin are shown in FIG. 13.

4) Data analysis

In gintonin-mediated LTP induction experiments, MC Rack (v.3.2.1.0, Multi Channel Systems) and an analyzing program (with aid from Dr. Tae-Sung Kim, department of medical-engineering Kyung-Hee University) using MatLab (v.7.0.1, The Mathworks inc.) was used to analyze the data (Moon et al., Neursci. Lett. 466, 114-119, 2009; Lee et al., J Neurosci. Res. 89, 96-107, 2011).

As is understood from the plot of filed excitatory postsynaptic potentials (fEPSPs) on Y-axis vs. time on X-axis in FIG. 13, theta burst stimulation (TBS) induced LTP in the slices in the absence of gintonin, but stronger LTP in gintonin-treated slice according to gintonin concentration.

FIG. 14 shows representative field traces (20 min) before TBS and 80 min after TBS. In the control (not treated with gintonin), no activated regional potentials were detected while regional potentials of gintonin-treated slices were increased in a concentration-dependent manner. FIG. 14 shows a summary of the total field excitatory postsynaptic potentials (total fEPSPs) which were recorded at 152.8+7.21% in the control, but increased to $180.90 \pm 7.53\%$ at gintonin 0.1 $\mu$g/ml, to $205.32 \pm 6.40\%$ at gintonin 1 $\mu$g/ml, and to 214.14+11.55% at gintonin 10 $\mu$g/ml, demonstrating that gintonin induces LTP in hippocampal slices (n=4, each) (see FIG. 15).

EXAMPLE 12: Effect of Gintonin on Long-Term Memory and Spatial Cognition

Activation of NMDA receptors in the central nervous system is associated with the induction of LTP, which, in turn, plays a pivotal role in enhancing learning and memory (Rezvani AH., In: Levin ED, Buccafusco JJ, editors. Animal Models of Cognitive Impairment. Boca Raton (FL): CRC Press; 2006. Chapter 4). Since, as illustrated above, gintonin was found to evoke NMDA receptor activation, a passive avoidance test and a Morris water maze test were conducted with mice to examine the effect of gintonin on memory.

12-(1) Examination of long-term memory by passive avoidance test

ICR male mice (25-28 g), purchased from Oriental Bio (Seoul, Korea), were bred according to the guideline of the Institutional Animal Care and Use Committee of Konkuk University, and subjected to passive avoidance test to examine memory maintenance impairment and the protection of gintonin against memory impairment according to the method of Yang et al. (Yang et al., Biol Pharm Bull 32, 1710-1715, 2009).

A passive avoidance test was performed in a training chamber which was divided into two compartments (20x20x20 cm in size, each) separated by an automatically moving guillotine door (5x5 cm): one compartment was lighted while the other remained dark where metal rods installed in a lattice pattern on the bottom so as to deliver an electrical foot shock (Gemini Avoidance System, San Diego, USA).

1) Learning session

For an acquisition trial, a mouse was first placed in the lighted compartment, facing away from the dark compartment and allowed to explore for 10 sec. After 10 sec, the guillotine door was opened and the mouse was allowed to explore freely. When the mouse entered the dark compartment with all four paws according to its habit, the guillotine door was closed, and a footshock (0.5 mA, 3 sec duration) was delivered. The latency time to enter the dark compartment was recorded (from the time the door was lifted). 120 min before experimentation, gintonin (25, 50, 100 mg/kg, *p.o*) was administered while tacrine (10 mg /kg, *p.o*) was used as a positive control. Memory impairment was induced by injecting scopolamine (0.9 mg/kg, *i.p.*) 30 min after gintonin or tacrine administration (Araujo et al., Prog Neuropsycopharmcol Biol Psychiatry 29, 411-422, 2005). For a negative control, a physiological saline was used. An experiment started 30 min after scopolamine administration, and the case where the mice did not enter the dark compartment for 180 sec was excluded.

2) Test session

24 Hours after the learning session, an experiment was performed without injecting gintonin. The mouse was positioned again in the lighted compartment. After 10 sec of acclimation, the latency time to enter the dark compartment was recorded (from the time the door was lifted).

By a passive avoidance test, the effect of gintonin on scopolarmin-induced long-term memory impairment was evaluated. The latency times obtained in the passive avoidance test are depicted in FIG. 16. No differences in latency time between scopolamine-administered groups were observed in the acquisition trial whereas gintonin increased the latency time reduced by scopolamine in a dose-dependent manner.

The latency time was recorded to be $23.07 \pm 1.95$ s in the group administered with scopolamine alone, and was increased to $78.24 \pm 7.83$ s, $169.22 \pm 12.97$ s, and $183.43 \pm 10.89$ s in the groups administered with gintonin at doses of 25, 50, and 100 mg/kg, respectively.

From the result, it was understood that gintonin can protect the brain nervous system from scolamine-caused long-term memory impairment.

12-(2). Examination of spatial cognition by Morris water maze test

The Morris water maze set up contained a round water pool (45 cm deep with a diameter of 90 cm) filled with milk-mixed water (22±1°C) to a depth of 30 cm. It was placed in a dark, sound-shielded room in which various visual cues were established. A white platform (6 cm in diameter, 29 cm in height) was inside the pool, with the top thereof 1 cm below the water surface in the center of one quadrant of the maze

On day 1, the mice were trained to swim for 60 sec. Subsequently, the training was conducted four times a day for four consecutive days, with the platform inside the pool. When the mouse reached the platform, it was allowed to sit on the platform only for 10 sec. If the failed in reaching the platform within 60 sec, it was guided to reach the platform and stay for 10 sec thereon. Afterwards, the mouse was returned back to the home cage and allowed to dry its body under an IR lamp. The time interval between trials was 30 sec.

The time to reach the hidden platform was recorded in each trial using a video camera-based Ethovision System (Nodulus, Wageningen, the Netherlands).

For each training trial, the mouse was placed into the pool at one quadrant at positions which were different day by day. One day after the final training trial, the mouse was evaluated. In this regard, after the platform was removed, the mouse was allowed to swim for 60 sec in search for the platform, and the time to swim in the quadrant where the platform was located was recorded. Gintonin (25, 50, or 100 mg/kg, *p.o.*) in physiological saline was administered 1 hr before test, every day. Tacrine (10 mg/kg, *p.o.*) was used as a positive control. Memory impairment was induced by scopolamine (0.9 mg/kg *i.p.*) 30 min after gintonin administration. For a negative control, only physiological saline was administered.

1) Latent time

The effects of gintonin at various concentrations (25, 50, and 100 mg/kg) on spatial cognition learning were measured by the water maze tests. The control administered with physiological saline was observed to quickly find out the position of the platform below the water surface, with the latent time remarkably decreasing as the round of the training increased (FIG. 17A). In contrast, only a small change was detected in the latent time of the scopolamine group over four days. In addition, the latent time of the scopolamine group was longer than that of the control during the training days (FIG. 17A). Gintonin was found to reduce the latent time extended by scopolamine significantly and in a dose-dependent manner (FIG. 17A).

Gintonin-treated groups steadily decreased in latent time over the time range from day 1 to day 4, demonstrating that gintonin significantly suppressed or protected against scopolamine-caused spatial cognition impairment.

2) Simple spatial reasoning

An examination was made of simple spatial reasoning. To this end, while the mice were allowed to swim in the absence of the rescue platform in the pool, the time period during which the mice swam in the quadrant where the rescue platform had been located was recorded.

As can be seen in FIG. 17B, the swimming time was detected to be $13.47\pm1.86$ s for the group administered with scopolamine alone, and increased to $21.33\pm3.13$ s, $24.53\pm3.91$ s, and $25.17\pm4.43$ s in the groups administered with gintonin at doses of 25, 50 and 100 mg/kg, respectively.

That is, the swimming time of the gintonin-treated groups was increased in a dose dependent manner, and was significantly longer, compared to that of the control, indicating that gintonin improved simple spatial reasoning (FIG. 17B).

EXAMPLE 13: Effect of Gintonin on Catecholamine Secretion of Rat Adrenal Gland

A catecholamine is a generic name for monoamines having a catechol and a side-chain amine, including dopamine, epinephrine, and norepinephrine, which are hormones secreted mostly from the central nervous system and the peripheral nervous system.

When secreted from the central nervous system, catecholamines function to maintain one's consciousness and make one's consciousness clear. In addition, catecholamines enable humans and animals to be awake and increase in concentration. A deficiency in the catecholamine levels of the central nervous system causes attention deficit hyperactivity disorder (ADHD) and depression. In addition, catecholamines contribute to body homeostasis against various external stresses.

Catecholamines secreted from the peripheral nervous system, such as an adrenal gland, are composed mainly of epinephrine, and are released to blood through the activation of G protein-coupled receptors present in chromaffin cells of adrenal medulla when the sympathetic nervous system is excited by, such as, an exercise, a stresse or a risk (Currie, Cell Mol Neurobiol. 8, 1201-1208, 2010). Released catecholamines promote the degradation of stored sugars and lipids to provide energy needed by the body in response to the exercise or stress, thereby playing an important role in exercise enhancement, adaptation to stress, and recovery from stress-caused fatigue. In addition, catecholamines act to constrict peripheral vessels under stress to maintain a blood pressure, and they thus can increase blood circulation, and are applicable to the prevention and treatment of hypotension (Purves, Dale, George J. Augustine, David Fitzpatrick, William C. Hall, Anthony-Samuel LaMantia, James O. McNamara, and Leonard E. White (2008). Neuroscience. 4th ed. Sinauer Associates. pp. 137-138).

In this Example, the effect of gintonin on the catecholamine secretion from rat adrenal gland was examined as follows.

13-(1). Preparation of experimental animals

Male rats (Sprague-Dawley, each weighing 200 to 300 g) was anesthetized by an intraperitoneal injection of thiopental sodium (50 mg/kgm), and the adrenal gland was excised from the rats according to the method of Wakade and Woo et al. (Wakade, J Physiol. 313, 463-480, 1981; Woo et al., Korean J Physiol Pharmacol., 12, 155-164, 2008).

Briefly, a cannula was inserted so as to perfuse the adrenal gland, as illustrated in FIG. 18. During the cannula insertion, heparin (400 IU/ml) was infused into the vena cava in order to prevent blood coagulation. Then, the adrenal gland was isolated from the body, and transferred to a leucite chamber which was kept at $37 \pm 1°C$.

13-(2). Perfusion of adrenal gland

The adrenal gland was perfused at a rate of 0.33 ml/min with the aid of a peristaltic pump (ISCO® pump, WIZ Co. U.S.A.). The perfusion solution was a Krebs-bicarbonate solution: (mM) NaCl, 118.4; KCl, 4.7; $CaCl_2$, 2.5; $MgCl_2$, 1.18; $NaHCO_3$, 25; $KH_2PO_4$, 1.2; glucose, 11.7.

The perfusion solution was continuously aerated with 95% $O_2$+5% $CO_2$ gas, and was maintained to have pH 7.4~7.5. In addition, the perfusion solution was supplemented with EDTA (10 mg/ml) and ascorbic acid (100 mg/ml) to prevent the oxidation of catecholamines (Woo et al., Korean J Physiol Pharmacol., 12, 155-164, 2008).

13-(3). Gintonin and drug administration

Gintonin (1~10 $\mu$g/ml) or cyclopiazonic acid (10 $\mu$M) was applied by perfusion for 4 min while acetylcholine (5.32 mM) was infused at a dose of 50 $\mu$l using a three-way stopcock.

13-(4). Collection of perfusate containing catecholamine

Prior to treatment with a material stimulating catecholamine secretion, the adrenal gland was left until the secretion of catecholamine in the absence of stimuli reached a constant level, that is, a background or basal level, which typically took 5~10 min. Then, the adrenal gland was perfused with gintonin or other drugs, and perfusates effluent from the adrenal gland were collected at regular intervals of 4 min, and the amount of catecholamines released by drug stimulation was calculated by subtracting the background or basal level from measurements (Woo et al., Korean J Physiol Pharmacol., 12, 155-164, 2008).

In order to examine the effect of gintonin on the spontaneous secretion of catecholamine and on the stimulus-induced secretion of catecholamine, the adrenal gland was perfused with a Krebs solution containing gintonin alone or in combination with other drugs, after which the perfusates were collected until the amount of secreted catecholamine reached the background level. The collected adrenal perfusates were stored in tubes maintained at 4°C.

13-(5). Quantitation of secreted catecholamine

Catecholamine in the perfusates was quantified using a fluorospectrophotometer (Kontron Co., Milano, Italy) according to the methods of Anton and Sayre, and Lim. In this regard, each perfusate amounted to 0.2 ml (Woo et al., Korean J Physiol Pharmacol., 12, 155-164, 2008).

13-(6). Statistical analysis

The differences between the control and treatment data of catecholamine were determined using Student's test and ANOVA test. A p-value of less than 0.05 was considered statistically significant.

As can be seen in FIG. 19, 15-min interval perfusion with gintonin stimulated the rat adrenal gland to continually secrete catecholamine in a dose-dependent manner.

Further, as is understood from the data of FIG. 20, pretreatment with 5.32 mM acetylcholine incapacitated the effect of 1 $\mu$g/ml gintonin on the secretion of catecholamine. At a level of 3 $\mu$g/ml, however, gintonin was observed to continually promote the secretion of catechlamine. When its amount was increased to 10 $\mu$g/ml, gintonin stimulates the secretion of catecholamine in the early stage, but could not induce the secretion since then. Therefore, acetylcholine-induced activation of muscarinic or nicotinic acetylcholine receptors suppressed reactivity with gintonin at 10 $\mu$g/ml; indicating that the receptors are in relation with cross desensitization with regard to the two ligands (FIG. 20).

From these results, it is expected that the gintonin of the present invention can promote the secretion of catecholamine from the adrenal gland, and thus is applicable to activating energy metabolism and nervous systems, and maintaining psychiatric concentration.

EXAMPLE 14: Gintonin-Induced Activation of Vascular $BK_{Ca}$ $K^+$ Channel

Large-conductance $Ca^{2+}$-activated $K^+$ channels ($BK_{Ca}$) are distributed over human and animal vascular smooth muscle cells, except for myocytes. They contributes to the regulation of vascular tone to enable vessels to dilate normally after vasoconstriction (Eichhorn et al., Naunyn-Schiemdeberg's Arch Pharmacol 376, 145-1551, 2007).

Dysfunction of $BK_{Ca}$ $K^+$ channels causes hypertension, ataxia, erectile dysfunction, and bladder dysfunction, the combination thereof which lead to urinary incontinence (Ledoux et al., Physiol. 21, 69-78, 2006).

During the depolarization-caused constriction of vascular smooth muscles, in addition, $BK_{Ca}$ $K^+$ channels are activated upon calcium influx to induce vasodilation after vasoconstriction, and the relaxation of penile corpus spongiosum and bladder smooth muscles (Eichhorn et al., Naunyn-Schiemdeberg's Arch Pharmacol 376, 145-1551, 2007). Hence, drugs to activate $BK_{Ca}$ $K^+$ channels ($BK_{Ca}$ $K^+$ channel opener) are applicable as therapeutics for hypertension, erectile dysfunction, and urinary incontinence (Ledoux et al., Physiol. 21, 69-78, 2006).

In this Example, $BK_{Ca}$ $K^+$ channel activity in *Xenopus* laevis oocytes was measured to examine whether gintonin activates vascular $BK_{Ca}$ $K^+$ channels, as follows.

14-(1). Oocyte preparation

*Xenopus laevis* frogs were obtained from Xenopus I (Ann Arbor, MI). Their care and handling were in accordance with the highest standards of institutional guidelines. To isolate oocytes, the frogs were operated on under anesthesia with an aerated solution of 3-aminobenzoic acid ethyl ester. Oocytes were separated by treatment with collagenase and agitation for two hours in a $Ca^{2+}$-free medium containing 82.5mM NaCl, 2 mM KCl, 1 mM $MgCl_2$, 5 mM HEPES, 2.5 mM sodium pyruvate, 100 units/ml penicillin and 100 $\mu$g/ml streptomycin.

Stage V-VI oocytes were collected and stored in ND96 (96 mM NaCl, 2 mM KCl, 1 mM $MgCl_2$, 1.8 mM $CaCl_2$, and 5 mM HEPES, pH 7.4) supplemented with 50 $\mu$g/ml gentamicin. This oocyte containing solution was maintained at 18°C with continuous gentle shaking while the ND96 medium was changed with a fresh one every day.

14-(2). Measurement of $BK_{Ca}$ $K^+$ Channel Activity

Two-electrode voltage-clamp recordings were obtained from individual oocytes placed in a small Plexiglas net chamber (5 ml). Electrophysiological experiments were performed using microelectrodes filled with 3 M KCl (resistance of 0.2-0.7 M$\Omega$) and an Oocyte Clamp amplifier (OC-725C; Warner Instrument, CT, USA) to record $BK_{Ca}$ $K^+$ channel activity at room temperature (Choi et al., Mol Cells 31, 133-140, 2011). For physiological recordings of $BK_{Ca}$ $K^+$ channel activity, oocytes were first perfused with a $Cl^-$ and $Ca^{2+}$-free solution (mM: 96 NaOH, 2 KOH, 8 Mg-gluconate, 5 HEPES, 5 EGTA, pH 7.4 with methanesulfonic acid) supplemented with 500 $\mu$M anthracene-9-carboxylic acid, a $Cl^-$ channel blocker, to block endogenous Cl-channels (Lu et al., J. Biol. Chem. 265, 16190-16194.1990). Then, the oocytes were clamped at -80 mV holding potential, followed by step depolarization from the holding potential to + 40 mV at regular intervals of 10 for 400 ms to record outward currents.

As can be seen in FIG. 21, gintonin activated $BK_{Ca}$ $K^+$ channels expressed in *Xenopus* oocytes in a dose-dependent manner, with an $ED_{50}$ detected to be $0.71 \pm 0.08$ $\mu$g/ml (FIG. 21B). In addition, gintonin was observed to activate $BK_{Ca}$ $K^+$ channels in a voltage-dependent manner, as elucidated in the current-voltage relationship (data not shown). Repetitive treatments of gintonin reduced $BK_{Ca}$ $K^+$ channel activity. That is, there occurred desensitization (data not shown).

Since the gintonin-induced activation of $BK_{Ca}$ $K^+$ channels is mediated through G protein-PLC-$IP_3$-$Ca^{2+}$ pathways by activation of LPA1 receptors endogenous to *Xenopus* oocytes (Kimura et al., J Biol Chem. 276, 15208-15215, 2001), LPA receptor antagonists were examined for ability to block the gintonin-induced activation of $BK_{Ca}$ $K^+$ channels.

As is understood from the data of FIG. 22A, treatment with Ki16425 (10 $\mu$M), known as an antagonist to LPA1 and LPA3 receptors (Ohta et al., Mol Pharmacol 64. 994-1005, 2003), reduced the gintonin-induced activation of $BK_{Ca}$ $K^+$ channels, demonstrating that LPA receptors mediate the gintonin-induced activation of $BK_{Ca}$ $K^+$ channels (FIG. 22B).

Moreover, an examination was made of the pathway through which gintonin signaling is mediated to $BK_{Ca}$ $K^+$ channel activation.

Treatment with the active PLC (phospholipase C) inhibitor U73122 (1 $\mu$M) blocked gintonin-induced $BK_{Ca}$ $K^+$ channel activation (FIG. 23A and 23C) whereas the inactive phospholipase C (PLC) inhibitor U73343 (1 $\mu$M) did not block gintonin-induced $BK_{Ca}$ $K^+$ channel activation (FIG. 23B and 23D). On the other hand, the activity of ginsenoside Rg3 was not blocked by the active PLC inhibitor U73122 (1 $\mu$M), indicating that the ginenoside Rg3 takes a signaling pathway different from that taken by gintonin (Choi et al., Mol Cells. 31, 133-140, 2011).

Likewise, the $IP_3$ receptor antagonist 2-APB (100 $\mu$M) blocked gintonin-induced $BK_{Ca}$ $K^+$ channel activation (FIGS. 24A and 24C). In addition, when treated with the intracellular calcium chelator BAPTA-AM, the Xenopus oocytes showed a reduction in $BK_{Ca}$ $K^+$ channel activation (FIGS. 24B and 24D). These data confirmed that gintonin induces the activation of $BK_{Ca}$ $K^+$ channels via the $PLC$-$IP_3$-$Ca^{2+}$ pathways whereas ginsenoside Rg3 takes a different pathway as its activity is not blocked by either 2-APB or BAPTA-AM (Choi et al., Mol Cells. 31, 133-140, 2011).

In addition, to investigate the involvement of protein kinase C(PKC) in the gintonin-induced activation of $BK_{Ca}$ $K^+$ channels, their activity was measured after the PKC activator PMA was applied in advance.

As can be seen in FIG. 25, pretreatment with PMA (1 $\mu$M) caused the gintonin-induced activation of $BK_{Ca}$ $K^+$ channels to disappear, but had no influences on the activity of ginsenoside Rg3, implying that gintonin and ginsenoside Rg3 exert their actions via different respective pathways, and that PKC is involved in mediating the action of gintonin.

EXAMPLE 15: Effect of Gintonin on Wound Healing in HUVEC

HUVECs have endogenous LPA1 and LPA3 receptors (Lin et al., BBRC 363, 1001-1008, 2007; Lin et al., Cellular Signalling 20, 1804-1814, 2008).

In this Example, an examination was made of the effect of gintonin on wound healing. For use in this examination, HUVECs were cultured in an M199 medium supplemented with heat-inactivated 20% FBS, 3 ng/ml basic fibroblast growth factor, 5 units/ml heparin, and streptomycin and penicillin in a 5% $CO_2$/95% $O_2$ incubator.

HUVEC ($2.5 \times 10^5$/well) were seeded to 24-well plates, and incubated for 24 hrs, and then for an additional 6 hrs in M199 supplemented with 1% FBS. Single wounds were made in the confluent monolayers by dragging a sterile 200-$\mu$l pipette tip across the monolayer to create a cell-free path. Floating cells were washed off twice with M199 supplemented with 1% FBS. The adherent cells were incubated with gintonin (30 $\mu$g/ml). Before and after treatment with gintonin, HUVECs in each well were observed and photographed (100x) using an inverted fluorescence microscope (AxioVert200; Carl Zeiss, Germany). Morphological comparison with non-treated cells was made.

In the assay for wound healing of gintonin, uncovered wound areas were measured using AxioVision 4 (Carl Zeiss, Germany) (FIG. 26A), and the time process of covering the scratch wound areas was monitored in the presence or absence of gintonin (FIG. 25B). In addition, uncovered scratch areas of gintonin-treated groups were compared with that of the control and expressed as % of that of the control (FIG. 26C and D).

EXAMPLE 16: Effect of Gintonin on Angiogenesis with HUVEC

16-(1). Effect of gintonin on HUVEC proliferation

BrdU can be incorporated into the newly synthesized DNA of replicating cells, substituting for thymidine during DNA replication (Porstmann et al. Immunol. Methods 82, 169-179, 1985).

In this Example, gintonin was examined for effect on cell growth. In this regard, HUVECs (human umbilical vein endothelial cells) were cultured with BrDU in the presence of gintonin, and analyzed for relative BrDU content by BrDU assay (Won et al., J Pharmacol Sci 108, 372-379, 2008; Chen et al., Cell Physiol Biochem 22, 307-314, 2008) using a BrDU cell proliferation ELISA kit (Roche, Germany).

Briefly, cells were seeded at a density of $3 \times 10^3$ cells/well into 96-well plates one day before treatment with gintonin. After 24 hrs of incubation, the cells were cultured for 6 hrs in a fresh M199 medium supplemented with 1% FBS. Again, the medium was changed to a fresh M199 medium supplemented with 1% FBS, after which the cells were incubated with gintonin for 24 hrs. For an additional 24 hrs, the cells were incubated with a BrDU labeling reagent. After medium was removed, the cells were washed with 10% FBS-supplemented M199, fixed with a fixative, and quantitatively analyzed for relative intracellular BrDU content using an anti-BrDU-peroxidase antibody and a luminometer (Veritas, Turner Biosystems, USA).

As a result, gintonin was observed to increase the incorporation of Br-DU into the DNA of HUVECs in a concentration-dependent manner (FIG. 27A).

16-(2). Effect of gintonin on cell migration

To measure the effect of gintonin on cell migration, a Boyden chamber (Neuro Probe Inc., Gaithersburg, MD, USA) assay was employed (Kim et al. Biol Pharm Bull 30: 1674-1679, 2007; Lee et al. Am J Physiol Cell Physiol 278:C612-C618, 2000).

In the Boyden chamber (48 wells) of two compartments separated by a collagen-coated polycarbonate microporous membrane (8-$\mu$m pores), gintonin in M199 (0.1% BSA contained) was placed onto the wells of lower compartment while a HUVEC suspension was added to the wells of the upper compartment ($5\times10^4$ cells/well). During incubation at 37°C for 70~80 min, the cells were allowed to migrate through the membrane into the lower compartment. Then, the chamber was disassembled, and the cells on the membrane was fixed and stained with Diff Quik (Sysmex, Kobe, Japan), and mounted on a slide. The cells that did not migrate were wiped off, and the cells which migrated were counted (magnification x200).

As can be seen in FIG. 27B, gintonin promoted cell migration in a concentration-dependent manner.

16-(3). Effect of gintonin on tubular formation

To examine the effect of gintonin on angiogenesis, formation of vascular tube-like structures on Matrigel was compared between the control and the gintonin-treated group (Kim et al. Biol Pharm Bull 30: 1674-1679).

Briefly, cells were incubated for 6 hrs in M199 (1% FBS). After trypsinization, the cells were suspended in M199 (1% FBS), and seeded ($2\times10^5$ cells/well) to 24-well plates coated with Matrigel (250 $\mu$l/well).

The cells were incubated at 37°C for 4 hrs in M199 (1% FBS) containing gintonin (30 $\mu$g/ml), and observed for tubular formation by inverted fluorescence microscopy (AxioVert200, Carl Zeiss). Microscopic images (magnification x100) showed that gintonin promoted tubular formation (FIG. 27C).

EXAMPLE 17: Inhibitory Activity of Gintonin against Chemotherapy-Caused Diarrhea and Mucositis

LPA receptors are distributed over the gastrointestinal tract, and their activation are reported to inhibit radiation or chemotherapy-induced apoptosis of gastrointestinal epithelial cells (Deng et al., Gastroenterology 123, 206-216, 2002; Deng et al., Gastroenterology 132, 1834-1851, 2007).

Meanwhile, when administered to humans, busulfan, a cancer drug for blood cancer, causes side effects including diarrhea and mucositis (Escaló$\Delta$n et al., Bone Marrow Transplant. 44, 89-96. 2009).

In this Example, an examination was made of the inhibitory activity of gintonin against chemotherapy-induced diarrhea and mucositis. For this, gintonin-treated mice were administered with busulfan, and observed for diarrhea and mucostitis.

Briefly, mice were orally administered at a dose of 100 mg/kg with gintonin 3 days before intraperitoneal injection with busulfan (40 mg/kg). For the control, physiological saline was used, instead of gintonin. Following busulfan injection, the mice were observed for 10 days for diarrhea and mucostitis.

Among a total of 15 busulfan-injected mice, 8 cases of diarrhea was detected, with a morbidity rate of 53.3%. In the gintonin-treated group, only 3 cases (20%) were observed to undergo diarrhea, so that gintonin reduced busulfan-caused diarrhea by about more than 30%.

As for perianal mucostitis, its onset was found in 7 of a total of 15 bufulfan-injected mice, with a morbidity of 46.7%, while only 2 cases of perianal mucostitis (13.3%) were detected, so that gintonin inhibited bufulfan-caused perianal mucostitis by 33.4% (Table 2, below).

TABLE 2

| Effect of Gintonin on Chemotherapy-Caused Diarrhea and Mucostitis | | |
| --- | --- | --- |
| Symptom | Busulfan | Gintonin+Busulfan |
| Diarrhea | 8/15 | 3/15 |
| Mucositis | 7/15 | 2/15 |

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

**Claims**

1.  A novel ligand, acting on a lysophosphatidic acid (LPA) receptor.

2.  The novel ligand of claim 1, being gintonin, a glycolipoprotein isolated from ginseng.

3.  The novel ligand of claim 2, wherein the gintonin has a structural protein composed of ginseng major latex-like protein (MLP151) and ribonuclease (RNAse)-like storage proteins.

4.  The novel ligand of claim 3, wherein the ginseng major latex-like protein (MLP151) comprises amino acid sequences set forth as SEQ ID NOS: 1 to 4.

5.  The novel ligand of claim 3, wherein the ginseng major latex-like protein (MLP151) has an amino acid sequence set forth as SEQ ID NO: 5, with three N-glycosylation sites.

6.  The novel ligand of claim 3, wherein the ginseng ribonuclease (RNAse)-like major storage protein comprises amino acid sequences set forth as SEQ ID NOS: 6 to 10.

7.  The novel ligand of claim 3, wherein the ginseng ribonuclease (RNAse)-like major storage protein has an amino acid sequence set forth as SEQ ID NO: 11.

8.  The novel ligand of claim 2, wherein the gintonin acts as an agonist of the LPA receptor.

9.  A method for identifying interaction between gintonin and an LPA receptor, comprising:

    (1) preparing large quantities of plasmids carrying respective LPA family receptor genes and an empty plasmid carrying none of them through amplification and purification (maxi-preparation);
    (2) verifying the expression of LPA receptors wherein B103 cells are transfected with haematoglutin (HA)-tagged LPA receptors, and then subjected to Western blotting analysis using an anti-HA primary antibody and a horseradish peroxidase (HRP)-conjugated secondary antibody to develop a color;
    (3) verifying the expression of LPA receptors wherein B1-3 cells are transfected with haematoglutin (HA)-tagged LPA receptors, and then subjected to confocal laser microscopy using an anti-HA antibody and a fluorescence dye Cy3-conjugated secondary antibody;
    (4) transfecting the empty plasmid and each of the plasmids carrying LPA family receptor genes into B103 cells;
    (5) treating the transfected B103 cells with trypsin (0.05% trypsin with EDTA, w/v) 2~3 days post-transfection, to give a cell suspension;
    (6) culturing the suspended B103 cells with Fura-2AM (2.5 $\mu$M); and
    (7) treating the suspended B103 cells with gintonin and quantifying a change in intracellular free $Ca^{2+}$ level in a cuvette by spectrofluorophotometry using Fura-2AM.

10. The method of claim 9, further comprising:

    (8) pre-treating the suspended B103 cells with LPA receptor antagonists and LPA receptor-mediated signaling-relevant drugs (e.g., pertussis toxin, PLC inhibitors, $IP_3$ receptor antagonists) to examine whether the cells decrease or increase in intracellular free $Ca^{2+}$, prior to treatment with gintonin;

(9) performing site-directed mutagenesis to identify an amino acid of LPA receptors with which gintonin interact to activate the LPA; and

(10) examining whether gintonin activates orphan GPCRs including GPR35 and GPR87, and free fatty acid GPCRs including GPR40, GPR41, GPR43 and GPR120, all known for activation by LPA.

11. A pharmaceutical composition for improving learning ability and memory by NMDA receptor activation and hippocampal LTP enhancement, comprising a ginseng-derived glycolipoprotein gintonin or a pharmaceutically acceptable salt thereof as an active ingredient.

12. A pharmaceutical composition for increasing resistance to stress and recovery from stress-induced fatigue, comprising a ginseng-derived glycolipoprotein gintonin or a pharmaceutically acceptable salt thereof as an active ingredient.

13. A pharmaceutical composition for wound healing, comprising a ginseng-derived glycolipoprotein gintonin or a pharmaceutically acceptable salt thereof as an active ingredient.

14. A pharmaceutical composition for prevention and treatment of a disease associated with vascular smooth muscle proliferation, comprising a ginseng-derived glycolipoprotein gintonin or a pharmaceutically acceptable salt thereof as an active ingredient.

15. The pharmaceutical composition of claim 14, wherein the disease associated with vascular smooth muscle proliferation is postoperative stenosis or recurrent stenosis.

16. A pharmaceutical composition for prevention and treatment of inflammation, comprising a ginseng-derived glycolipoprotein gintonin or a pharmaceutically acceptable salt thereof as an active ingredient.

17. The pharmaceutical composition of any one of claims 11 to 16, wherein the gintonin is sourced from roots, stems, and leaves of ginseng selected from among fresh ginseng, white ginseng, red ginseng, artificially sown but wild-grown ginseng, artificially bred ginseng, and wild ginseng.

FIG. 1

FIG. 2

FIG. 3

A

B

C

FIG. 4

FIG. 5

FIG. 6

FIG. 7

FIG. 8

FIG. 9

FIG. 10

FIG. 11

FIG. 12

FIG. 13

Control                                          GT −0.1 μg/ml

20 min            80 min            20 min            80 min

GT −1 μg/ml                                       GT −10 μg/ml

20 min            80 min            20 min            80 min

FIG. 14

FIG. 15

FIG. 16

FIG. 17a

FIG. 17b

FIG. 18

FIG. 19

FIG. 20

A

B

FIG. 21

**FIG. 22**

FIG. 23

FIG. 24

A

B

FIG. 25

FIG. 26

FIG. 27

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020100052913

**Non-patent literature cited in the description**

- **TYLER.** *J. Pharm. Technol.,* 1995, vol. 11, 214-220
- **SHIBATA et al.** *Tetraheadron Letters,* 1962, vol. 1239-124, 1963
- **WAGNER-JAUREGG ; ROTH.** *Pharm Acta Helv,* 1962, vol. 37, 352-357
- **NAH.** *Kor. J. Ginseng Sci.,* 1997, vol. 21, 1-12
- **PYO et al.** *J Ginseng Res,* 2011, vol. 35, 92-103
- **BERRIDGE et al.** *Natuare,* 1998, vol. 395, 645-648
- **BERRIDGE et al.** *Nature,* 1998, vol. 395, 645-648
- **OKUDAIRA et al.** *Biochimie,* 2010, vol. 92, 698-706
- **AOKI.** *Seminars Cell & Dev. Biol.,* 2004, vol. 15, 477-489
- **PAGES et al.** *Prostaglandins,* 2001, vol. 64, 1-10
- **CROSET et al.** *Biochem J,* 2000, vol. 345, 61-67
- **JALINK et al.** *Biochem J.,* 1995, vol. 307, 609-615
- **PILQUIL et al.** *Prostaglandins,* 2001, vol. 64, 83-92
- **BRINDLEY ; PILQUIL.** *J Lipid Res.,* 2009, vol. 50, 225-230
- **DENG et al.** *Gastroenterology,* 2007, vol. 132, 1834-1851
- **CHUN J et al.** *Pharmacol Rev.,* 2010, vol. 62, 579-587
- **AN et al.** *J Biol Chem,* 1998, vol. 283, 7906-7910
- **SKOURA ; HLA.** *J Lipid Res.,* 2009, vol. 50, S293-S298
- **TOMAN ; SPIEGEL.** *Neurochemical Research,* 2002, vol. 27, 619-627
- **TIGYI.** *Br J Pharmacol,* 2010, vol. 161, 241-270
- **YOSHIDA ; UEDA.** *Jpn J Pharmacol,* 2001, vol. 87, 104-109
- **DUBBIN et al.** *J Neurosci,* 2010, vol. 30, 7300-7309
- **YANAGIDA et al.** *J Biol Chem,* 2009, vol. 284, 17731-17741
- **CHOI et al.** *Biochim Biophys Acta,* 2008, vol. 1781, 531-539
- **CHOI et al.** *Biochemica Biophysica Acta,* 2008, vol. 1781, 531-539
- **YE X.** *Hum Reprod Update.,* 2008, vol. 14, 519-536
- **ONO et al.** *Mol. Cell Proteomics Sci.,* 2006, vol. 5, 1338-1347
- **GAO et al.** *Mol. Cell Proteomics Sci.,* 2008, vol. 7, 2399-2409
- **NAM et al.** *Proteomics,* 2003, vol. 3, 2351-2367
- **KIM et al.** *J Plant Physiol.,* 2004, vol. 161, 837-845
- **ISHII et al.** *Mol Pharmacol,* 2000, vol. 58, 895-902
- **LEE et al.** *J Biol Chem,* 2006, vol. 281, 23589-23597
- **BANDOH et al.** *J Biol Chem,* 1999, vol. 274, 27776-27785
- **IM et al.** *Mol Pharmacol,* 1999, vol. 57, 753-759
- **JØRGENSEN N.K. ; PETRSON S.F. ; HOFFMAN E.K.** *Am J Physiol Cell Physiol,* 1999, vol. 276, 26-37
- **HOUNSELL, E.F. ; DAVIES, M.J. ; SMITH, K.D.** Protein protocol handbood. Humanna press,, 1997, 803-804
- **GRYNKIEWICZ, G. ; M. POENIE ; R. Y. TSIEN.** *J Biol Chem,* 1985, vol. 260, 3440-3450
- **CONTOS et al.** *Mol Pharmcol,* 2000, vol. 58, 1188-1196
- **KIMURA et al.** *J Biol Chem.,* 2001, vol. 276, 15208-15215
- **OKA et al.** *Biochem Biophys Res Commun.,* 2010, vol. 395, 232-237
- **TABATA et al.** *Biochem Biophys Res Commun.,* 2007, vol. 363, 861-866
- **HIRASAWA et al.** *Biol Pharm Bull.,* 2008, vol. 31, 1847-1851
- **VALENTINE et al.** *Biochim Biophys Acta,* 2008, vol. 1780, 597-605

- **NAH et al.** *CNS Drug Rev.,* 2007, vol. 13, 381-404
- **HOUNSELL, E.F. ; DAVIES, M.J. ; SMITH, K.D.** Protein protocol handbood. Humanna press, 1997, 803-804
- **OHTA et al.** *Mol Pharmacol,* 2003, vol. 64, 994-1005
- **AN et al.** *Mol Pharmacol,* 1998, vol. 54, 881-886
- **URS et al.** *J Biol Chem.,* 2008, vol. 283, 5249-5257
- **VALENTINE et al.** *J. Biol. Chem,* 2008, vol. 283, 12175-12187
- **VALENTINE et al.** *J. Biol. Chem.,* 2008, vol. 283, 12175-12187
- **DINGLEDINE et al.** *Pharmacol. Rev.,* 1999, vol. 51, 7-61
- **CULL-CANDY et al.** *Curr. Opin. Neurobiol.,* 2001, vol. 11, 327-335
- **PAOLETTI ; NEYTON.** *Curr Opin Pharmacol,* 2007, vol. 7, 39-47
- **PURVES, DALE ; GEORGE J. AUGUSTINE ; DAVID FITZPATRICK ; WILLIAM C. HALL ; ANTHONY-SAMUEL LAMANTIA ; JAMES O. MCNAMARA ; LEONARD E. WHITE.** Neuroscience. Sinauer Associates, 191-195
- **ZHENG et al.** *J Neurosci.,* 1997, vol. 17, 8676-8686
- **CHANG ; KUO.** *J Neurosci.,* 2008, vol. 28, 1546-1556
- **URUSHIHARA et al.** *J Biol Chem,* 1992, vol. 267, 11697-11700
- **ZHENG et al.** *J Neuroscience,* 1997, vol. 15, 8676-8686
- **LIA et al.** *Mol Pharmaocol,* 2001, vol. 59, 960-964
- **LU et al.** *Nature neuroscience,* 1999, vol. 2, 331-338
- **TSAI et al.** *EMBO J,* 1999, vol. 18, 109-118
- **PETRONE et al.** *EMBO J,* 2003, vol. 22, 4121-4131
- **COOKE ; BLISS.** *Brain,* 2006, vol. 129, 1659-1673
- **BLISS ; COLLINGRIDGE.** *Nature,* 2003, vol. 361, 31-39
- **MOON et al.** *Neursci. Lett.,* 2009, vol. 466, 114-119
- **LEE et al.** *J Neurosci. Res.,* 2011, vol. 89, 96-107
- **STEIDL et al.** *Brain Res.,* 2006, vol. 1096, 70-84
- **LELONG ; REBEL.** *J Pharmacol Toxicol Methods.,* 1998, vol. 39, 203-210
- **STOPPINI et al.** *J Neurosci Methods,* 1991, vol. 37, 173-182
- **SHIMONO et al.** *Neural Plast.,* 2002, vol. 9 (4), 249-254
- **REZVANI AH.** Animal Models of Cognitive Impairment. CRC Press, 2006
- **YANG et al.** *Biol Pharm Bull,* 2009, vol. 32, 1710-1715
- **ARAUJO et al.** *Prog Neuropsycopharmcol Biol Psychiatry,* 2005, vol. 29, 411-422
- **CURRIE.** *Cell Mol Neurobiol.,* 2010, vol. 8, 1201-1208
- **PURVES ; DALE ; GEORGE J. AUGUSTINE ; DAVID FITZPATRICK ; WILLIAM C. HALL ; ANTHONY-SAMUEL LAMANTIA ; JAMES O. MCNAMARA ; LEONARD E. WHITE.** Neuroscience. Sinauer Associates, 2008, 137-138
- **WAKADE.** *J Physiol.,* 1981, vol. 313, 463-480
- **WOO et al.** *Korean J Physiol Pharmacol.,* 2008, vol. 12, 155-164
- **EICHHORN et al.** *Naunyn-Schiemdeberg's Arch Pharmacol,* 2007, vol. 376, 145-1551
- **LEDOUX et al.** *Physiol.,* 2006, vol. 21, 69-78
- **CHOI et al.** *Mol Cells,* 2011, vol. 31, 133-140
- **LIN et al.** *BBRC,* 2007, vol. 363, 1001-1008
- **LIN et al.** *Cellular Signalling,* 2008, vol. 20, 1804-1814
- **PORSTMANN et al.** *Immunol. Methods,* 1985, vol. 82, 169-179
- **WON et al.** *J Pharmacol Sci,* 2008, vol. 108, 372-379
- **CHEN et al.** *Cell Physiol Biochem,* 2008, vol. 22, 307-314
- **KIM et al.** *Biol Pharm Bull,* 2007, vol. 30, 1674-1679
- **LEE et al.** *Am J Physiol Cell Physiol,* 2000, vol. 278, C612-C618
- **KIM et al.** *Biol Pharm Bull,* vol. 30, 1674-1679
- **DENG et al.** *Gastroenterology,* 2002, vol. 123, 206-216
- **ESCALÓⲆN et al.** *Bone Marrow Transplant.,* 2009, vol. 44, 89-96